# EUROPÄISCHE PATENTSCHRIFT

(11) **EP 0 787 731 B1**
(45) Veröffentlichungstag und Bekanntmachung des Hinweises auf die Patenterteilung: **07.08.2002**
(21) Anmeldenummer: 97810031.1
(22) Anmeldetag: 22.01.1997
(51) Int. Cl.: C07D 487/04, C08F 2/42, C08F 20/20, C08G 18/32

(54) **Polymerisierbare Diketopyrrolopyrrole und damit hergestellte Polymere**
Polymerizable diketopyrrolopyrroles and polymers thereof
Dicétopyrrolopyrroles et leurs polymères

(30) Priorität: 30.01.1996 CH 22796
(43) Veröffentlichungstag der Anmeldung: 06.08.1997
(73) Patentinhaber: Ciba Specialty Chemicals Holding Inc., 4057 Basel (CH)
(72) Erfinder: Eldin, Sameer Hosam, 1784 Courtepin (CH); Iqbal, Abul, 1732 Arconciel (CH)

(56) Entgegenhaltungen:
- EP-A- 0 133 156
- EP-A- 0 337 951
- EP-A- 0 648 770
- EP-A- 0 673 940
- EP-A- 0 704 497
- EP-A- 0 718 697
- J. AM. CHEM. SOC., Bd. 115, Nr. 25, 1993, Seiten 11735-11743, XP000652156 W.-K. CHAN ET AL.: "Rational Design of Multifunctional Polymers"

## Beschreibung

Die vorliegende Erfindung betrifft neue Diketopyrrolopyrrole mit zur Polyreaktion befähigten reaktiven Gruppen und damit hergestellte Polymere.

Die nunmehr auch in der Literatur z.B. in Colour Index, als DPP-Pigmente bezeichneten und seit einigen Jahren bekannten und als wertvoll bewährten Diketopyrrolopyrrol-Pigmente sind u.a. in US-Patent 4 415 685 und US-Patent 4 579 949 beschrieben.

In EP-A 337 951 werden farbige Polymermikropartikel beschrieben, die durch Einpolymerisieren von zur Polymerisation befähigten reaktiven Gruppen enthaltenden Pigmentderivaten in verschiedenartigen Polymeren erhalten werden können. Es werden dabei Derivate der verschiedensten Pigmentklassen erwähnt, darunter auch DPP-Derivate, eines davon, ein an beiden Stickstoffatomen durch eine Ethylmethacrylatgruppe substituiertes 1,4-Diketo-3,6-diphenylpyrrolo[3,4-c]pyrrol, spezifisch.

Es hat sich jedoch erwiesen, dass die Einpolymerisierung dieser Verbindung nicht zufriedenstellend erfolgt.

Es ist nun gefunden worden, dass durch Einführung spezieller, langkettiger Reaktivgruppen, doch DPP-Chromophore erhalten werden können, die überraschend leicht zu oder mit Polymeren umgesetzt werden können, sei es direkt durch Homo- oder Copolymerisation oder auch durch Aufpfropfung auf bereits vorgebildeten Homo- oder Copolymeren.

Die vorliegende Erfindung betrifft demnach 1,4-Diketopyrrolopyrrole der Formel 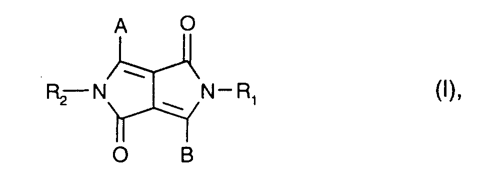 worin A und B unabhängig voneinander für eine Gruppe der Formel 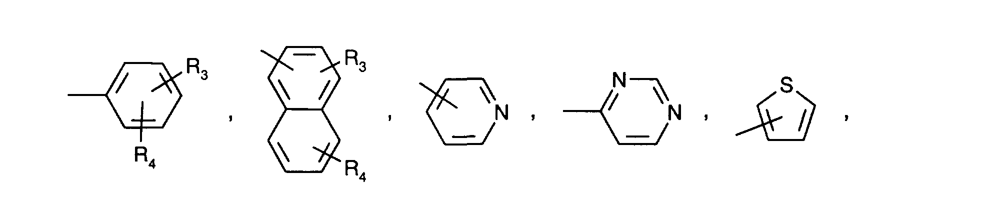 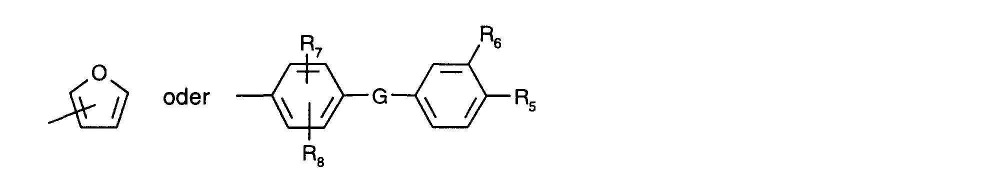 stehen, worin
R₃ und R₄ unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, C₁-C₁₈-Alkoxycarbonyl, C₁-C₁₈-Alkylaminocarbonyl, -CN, -NO₂, Trifluormethyl, C₅-C₆-Cycloalkyl, -C=N-(C₁-C₁₈-Alkyl), 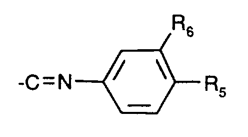 Imidazolyl, Pyrrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,
G -CH₂-, -CH(CH₃)-, -C(CH₃)₂-, -CH=N-, -N=N-, -O-, -S-, -SO-, -SO₂-, -CONH- oder -NR₉- ist,
R₅ und R₆ unabhängig voneinander Wasserstoff, Halogen, C₁-C₆-Alkyl, C₁-C₁₈-Alkoxy oder -CN sind, R₇ und R₈ unabhängig voneinander Wasserstoff, Halogen oder C₁-C₆- Alkyl und R₉ Wasserstoff oder C₁-C₆-Alkyl bedeuten,
R₁ einen eine zur Polyreaktion befähigte reaktive Gruppe aufweisenden Rest mit mindestens 4 C-Atomen und R₂ C₁-C₆-Alkyl, 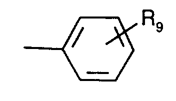 oder R₁ sind.

Unter zur Polyreaktion befähigten reaktiven Gruppen versteht man z.B. zur Polymerisation befähigte Gruppen, wie z.B. Acrylatreste, oder zur Polykondensation befähigte Gruppen, wie z.B. Hydroxy- oder Säurechloridgruppen, oder auch zur Polyaddition befähigte Gruppen, wie z.B. Hydroxy- oder Isocyanatgruppen.

Vorzugsweise bedeutet
R₁ einen Rest der Formel

-(CH₂)ₘ-CH=CH-(CH₂)ₙ-CH₃ (II)

oder

-(Y)ₚ-X-(Z)ᵣ-Q (III),

worin m und n unabhängig voneinander eine ganze Zahl zwischen Null und 12 sind, mit der Bedingung, dass die Summe m + n mindestens 4 ist, und p und r unabhängig voneinander Null oder 1 bedeuten,
X ununterbrochenes oder ein- oder mehrfach durch -O- und/oder -S-, -NH-, Phenylen, -COO-, -CONH-, 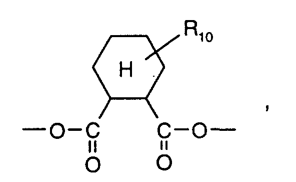 worin R₁₀ Wasserstoff oder Methyl ist, unterbrochenes C₄-C₁₈-Alkylen ist, 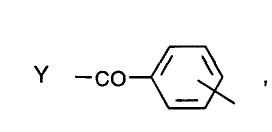 -Si(Cl)₂-, -Si(OC₂H₅)₂-, -Si(OCOCH₃)₂-, -CH₂-CH(OH)- oder -CH(CN)- und
Z -O-, -NH-, -COO-, Phenylen, 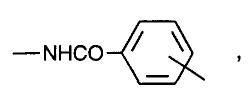 -Si(Cl)₂-, -Si(OC₂H₅)₂ -, -Si(OCOCH₃)₂-
bedeuten,
Q -OH, -NH₂, Glycidyl, 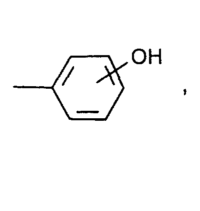 -CHO, -NCO, -CH=CH₂, -C(CH₃)=CH₂, -CO-CH=CH₂, -CO-C(CH₃)=CH₂, C₅-C₇-Cycloalkenyl, 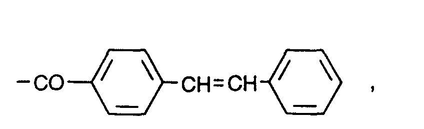 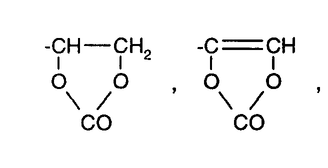 -CONHR₁₁, -COOR₁₁, -COR₁₁, worin R₁₁ Wasserstoff oder C₁-C₆-Alkyl ist, 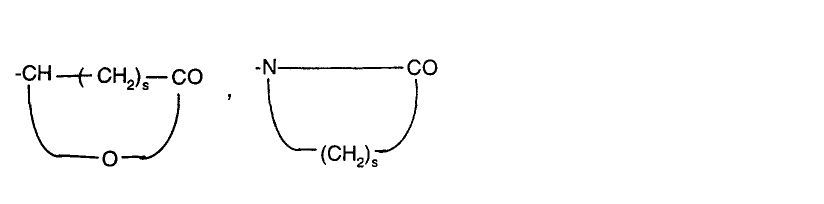 wobei s eine ganze Zahl von 1 bis 6 wie 1, 2, 3, 4, 5 und 6 ist, bedeutet.

Bedeuten etwaige Substitutenten Halogen, dann handelt es sich im allgemeinen um lod, Fluor, Brom oder Chlor, bevorzugt Brom oder Chlor, besonders bevorzugt Chlor;
der C₁-C₄-Alkyl-Rest steht für Methyl, Ethyl, n-Propyl, Isopropyl, n-Butyl, sec.-Butyl, i-Butyl, tert.-Butyl;
bei C₁-C₆-Alkyl handelt es sich beispielsweise um Methyl, Ethyl, n-Propyl, Isopropyl, n-Butyl, sec.-Butyl, i-Butyl, tert.-Butyl, n-Amyl, tert.-Amyl, Hexyl;
der C₁-C₁₈-Alkyl-Rest bedeutet beispielsweise Methyl, Ethyl, n-Propyl, Isopropyl, n-Butyl, sec.-Butyl, i-Butyl, tert.-Butyl, n-Amyl, tert.-Amyl, Hexyl, Heptyl, Octyl, 2-Ethylhexyl, Nonyl, Decyl, Dodecyl, Tetradecyl, Hexadecyl oder Octadecyl;
C₄-C₁₈-Alkylen, bevorzugt die linearen Vertreter, kann beispielsweise -(CH₂)₄-, -(CH₂)₅-, -(CH₂)₆-, -(CH₂)₇-,-(CH₂)₈-, -(CH₂)₉-,-(CH₂)₁₀-, -(CH₂)₁₁-, -(CH₂)₁₂-,-(CH₂)₁₃-, -(CH₂)₁₄-,-(CH₂)₁₅-, -(CH₂)₁₆-,-(CH₂)₁₇-, -(CH₂)₁₈- bedeuten, bevorzugt C₄-C₁₂-Alkylen wie -(CH₂)₄-, -(CH₂)₅-, -(CH₂)₆-, -(CH₂)₇-,-(CH₂)₈-, -(CH₂)₉-,-(CH₂)₁₀-, -(CH₂)₁₁-, -(CH₂)₁₂-;
C₁-C₁₈-Alkoxy bedeutet, auch in C₁-C₁₈-Alkoxycarbonyl, z.B. Methoxy, Ethoxy, n-Propoxy, Isopropoxy, Butyloxy, Hexyloxy, Decyloxy, Dodecyloxy, Hexadecyloxy oder Octadecyloxy, bevorzugt C₁-C₆-Alkoxy wie Methoxy, Ethoxy, n-Propoxy, Isopropoxy, Butyloxy, Hexyloxy;
C₁-C₁₈-Alkylmercapto steht beispielsweise für Methylmercapto, Ethylmercapto, Propylmercapto, Butylmercapto, Octylmercapto, Decylmercapto, Hexadecylmercapto oder Octadecylmercapto;
C₁-C₁₈-Alkylamino bedeutet, auch in C₁-C₁₈-Alkylaminocarbonyl, z.B. Methylamino, Ethylamino, Propylamino, Hexylamino, Decylamino, Hexadecylamino oder Octadecylamino, bevorzugt C₁-C₆-Alkylamin wie Methylamino, Ethylamino, Propylamino, Hexylamino.

C₅-C₆-Cycloalkyl steht z.B. für Cyclopentyl oder Cyclohexyl, insbesondere für Cyclohexyl.

C₅-C₇-Cycloalkenyl bedeutet mono- oder bicyclisches Cycloalkenyl, wie z.B. Cyclopentenyl, Cyclohexenyl oder Norbornenyl.

Einige Beispiele für R₁ als Rest der Formel III sind

-(CH₂)₆-OH,

-(CH₂)₁₀-OH,

-(CH₂)₁₁-OH,

-(CH₂)₆-OCO-CH=CH₂,

-(CH₂)₆-OCO-C(CH₃)=CH₂,

-(CH₂)₁₀-OCO-CH=CH₂,

-(CH₂)₁₀-OCO-C(CH₃)=CH₂,

-(CH₂)₁₁-OCO-CH=CH₂,

-(CH₂)₁₁-OCO-C(CH₃)=CH₂,

-(CH₂)₂-O-(CH₂)₂-O-(CH₂)₂-OH

-(CH₂)₂-O-(CH₂)₂-O-(CH₂)₂-O-CO-CH=CH₂

-(CH₂)₂-O-(CH₂)₂-O-(CH₂)₂-O-CO-C(CH₃)=CH₂

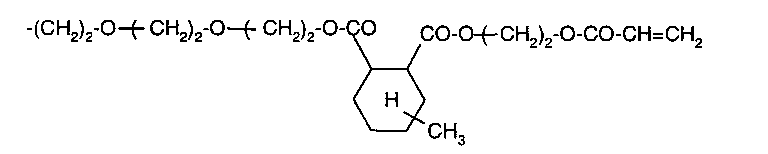

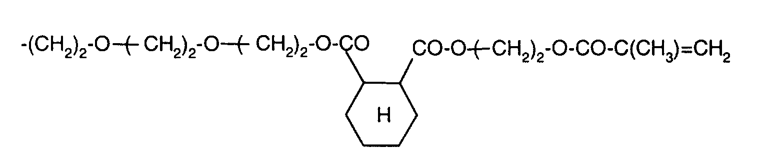

-(CH₂)₃-S-(CH₂)₂-OH

-(CH₂)₃-S-(CH₂)₆-OH

-(CH₂)₃-S-(CH₂)₂-COOH

-(CH₂)₃-S-(CH₂)₆-COOH

-(CH₂)₃-S-(CH₂)₂-NH₂

-(CH₂)₃-S-(CH₂)₆-NH₂

-(CH₂)₃-S-(CH₂)₂-O-(CH₂)₂-O-(CH₂)₂-OH

-(CH₂)₃-S-(CH₂)₂-O-(CH₂)₂-O-(CH₂)₂-NH₂

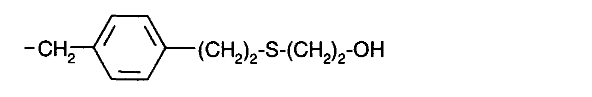

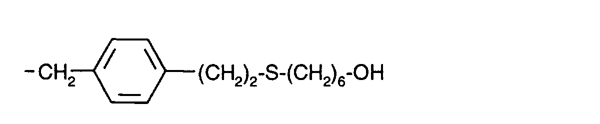

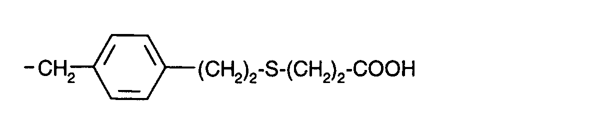

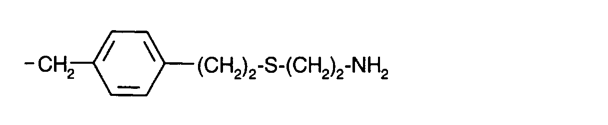

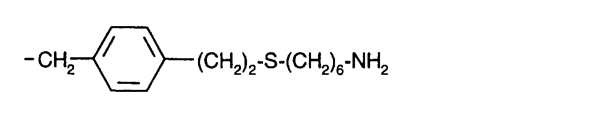

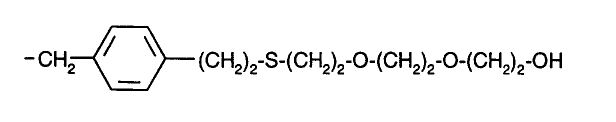

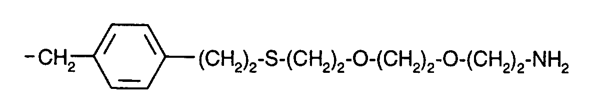

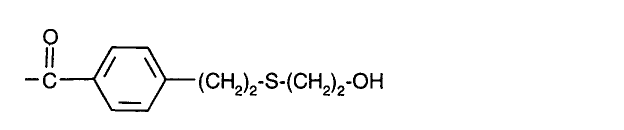

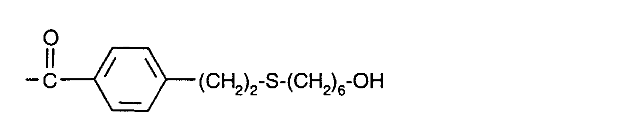

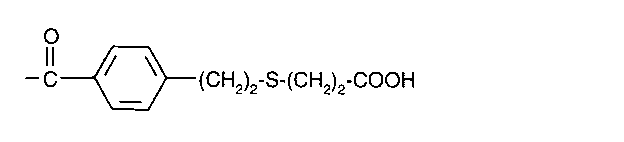

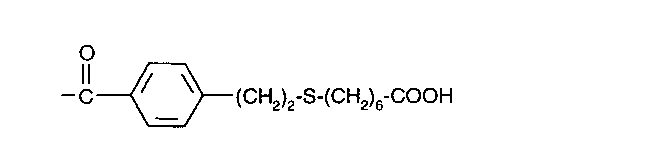

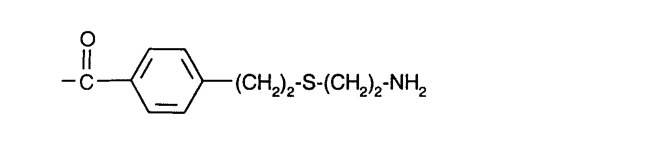

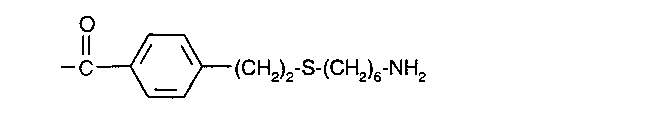

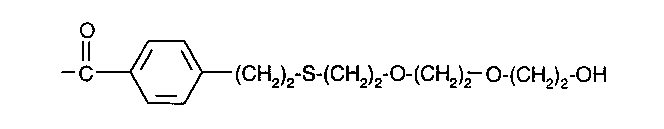

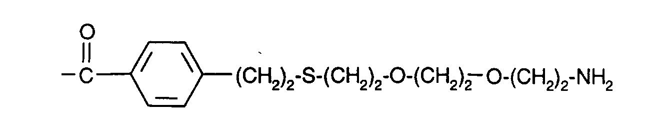

-(CH₂)₆-O-Si(Cl₂)-CH=CH₂

-(CH₂)₆-O-Si(OC₂H₅)₂-CH=CH₂

-(CH₂)₆-O-Si(O-COCH₃)₂-CH=CH₂

-Si(Cl)₂-(CH₂)₂-S-(CH₂)₂-OH

-Si(Cl)₂-(CH₂)₂-S-(CH₂)₆-OH

-Si(Cl)₂-(CH₂)₂-S-(CH₂)₂-COOH

-Si(Cl)₂-(CH₂)₂-S-(CH₂)₂-NH₂

-Si(Cl)₂-(CH₂)₂-S-(CH₂)₆-NH₂

-Si(Cl)₂-(CH₂)₂-S-(CH₂CH₂O)₂-CH₂CH₂OH

-Si(Cl)₂-(CH₂)₂-S-(CH₂CH₂O)₂-CH₂CH₂NH₂

-Si(OC₂H₅)₂-(CH₂)₂-S-(CH₂)₂-OH

-Si(OC₂H₅)₂-(CH₂)₂-S-(CH₂)₆-OH

-Si(OC₂H₅)₂-(CH₂)₂-S-(CH₂)₂-COOH

-Si(OC₂H₅)₂-(CH₂)₂-S-(CH₂)₆-COOH

-Si(OC₂H₅)₂-(CH₂)₂-S-(CH₂)₂-NH₂

-Si(OC₂H₅)₂-(CH₂)₂-S-(CH₂)₆-NH₂

-Si(OC₂H₅)₂-(CH₂)₂-S-(CH₂CH₂O)₂-CH₂CH₂OH

-Si(OC₂H₅)₂-(CH₂)₂-S-(CH₂CH₂O)₂-CH₂CH₂NH₂

-Si(OCOCH₃)₂-(CH₂)₂-S-(CH₂)₂-OH

-Si(OCOCH₃)₂-(CH₂)₂-S-(CH₂)₆-OH

-Si(OCOCH₃)₂-(CH₂)₂-S-(CH₂)₂-COOH

-Si(OCOCH₃)₂-(CH₂)₂-S-(CH₂)₆-COOH

-Si(OCOCH₃)₂-(CH₂)₂-S-(CH₂)₂-NH₂

-Si(OCOCH₃)₂-(CH₂)₂-S-(CH₂)₆-NH₂

-Si(OCOCH₃)₂-(CH₂)₂-S-(CH₂CH₂O)₂-CH₂CH₂OH

-Si(OCOCH₃)₂-(CH₂)₂-S-(CH₂CH₂O)₂-CH₂CH₂NH₂

-CH₂CH(OH)-CH₂-S-(CH₂)₆-COOH

-CH₂CH(OH)-CH₂-S-(CH₂)₆-OH

-CH₂CH(OH)-CH₂-S-(CH₂CH₂O)₂-CH₂CH₂OH

-CH₂CH(OH)-CH₂-S-(CH₂CH₂O)₂-CH₂CH₂COOH

-CH₂CH(OH)-CH₂-NH-(CH₂)₆-COOH

-CH₂CH(OH)-CH₂-NH-(CH₂)₆-OH

-CH₂CH(OH)-CH₂-NH-(CH₂CH₂O)₂-CH₂CH₂OH

-CH₂-CONH-(CH₂)₆-OH

-CH₂-CONH-(CH₂)₆-COOH

-CH₂-CONH-(CH₂CH₂O)₂-CH₂CH₂OH

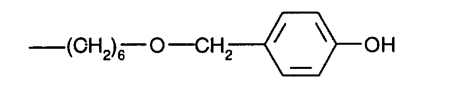

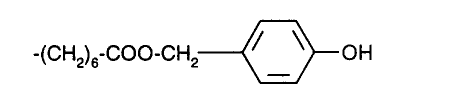

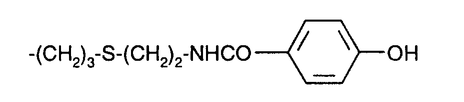

-CH(CN)-(CH₂)₆-OH

-CH(CN)-(CH₂CH₂O)₂-CH₂CH₂OH

-(CH₂)₆-O-CH₂CH₂O-CH=CH₂

-(CH₂)₆-O-CH₂-CH=CH₂

-(CH₂)₆-O-CH=CH₂

-(CH₂)₆-O-CH₂-CO-CH=CH₂

-(CH₂)₆-O-CO-CH=CH₂

-(CH₂)₆-O-(CH₂)₂-NHCO-CH=CH₂

-(CH₂)₆-O-CH₂-COO-CH=CH₂

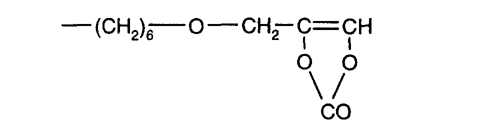

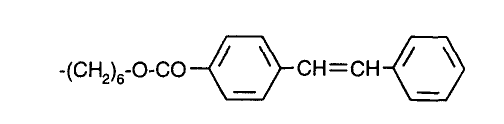

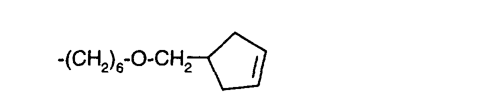

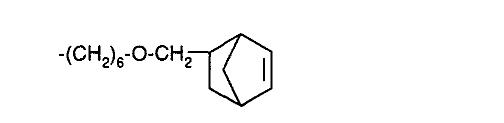

-(CH₂)₆-O-CH₂-CHO

-(CH₂)₆-O-CH₂-NCO

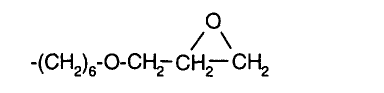

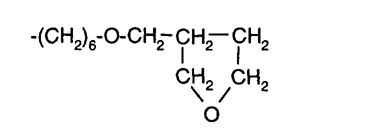

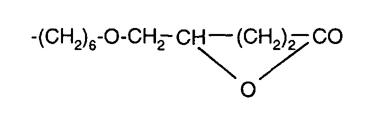

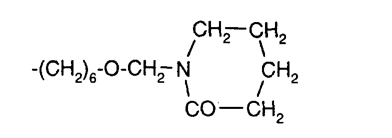

-(CH₂)₃-S-(CH₂)₂-CO-O-CO-CH₃.

Von besonderem Interesse sind die erfindungsgemässen Diketopyrrolopyrrole der Formel I, worin A und B unabhängig voneinander eine Gruppe der Formel 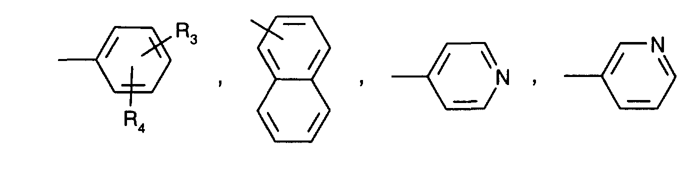 oder 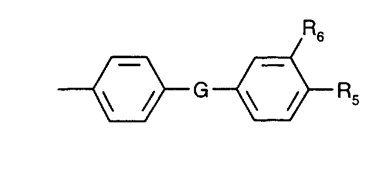 sind, worin R₃ und R₄ unabhängig voneinander Wasserstoff, Halogen wie Chlor oder Brom, C₁-C₄-Alkyl, C₁-C₆-Alkoxy, C₁-C₆-Alkylamino oder CN bedeuten,
G -O-, -NR₉-, -N=N- oder -SO₂- ist,
R₅ und R₆ Wasserstoff R₉ Wasserstoff, Methyl oder Ethyl bedeuten,
und insbesondere jene, worin in der Formel I A und B gleich sind und eine Gruppe der Formel 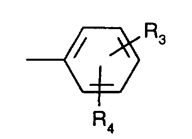 bedeuten, worin R₃ und R₄ unabhängig voneinander Wasserstoff, Methyl, tert.-Butyl, Chlor, Brom oder CN sind. R₄ ist bevorzugt Wasserstoff.

Von besonderer Bedeutung sind jene erfindungsgemässen Diketopyrrolopyrrole der Formel I, worin
R₁ einen Rest der Formel

-X-(O)ᵣ-Q (IV)

bedeutet, worin
X ununterbrochenes oder 1, 2 oder 3 Mal durch O und/oder einmal durch -S-, -NH-, 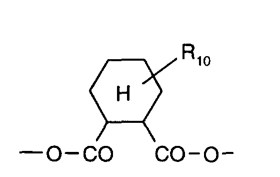 unterbrochenes C₄-C₁₂-Alkylen ist,
r und R₁₀ die oben angegebene Bedeutung haben, und
Q -OH; -CH=CH₂, -C(CH₃)=CH₂, -CO-CH=CH₂ oder -CO-C(CH₃)=CH₂ bedeutet.

X bedeutet vorzugsweise -(CH₂)_{q}-, wobei q eine ganze Zahl zwischen 6 und 12 wie 6, 7, 8, 9 ,10, 11 und 12 sein kann,

-(CH₂CH₂O)₂-CH₂CH₂-

oder 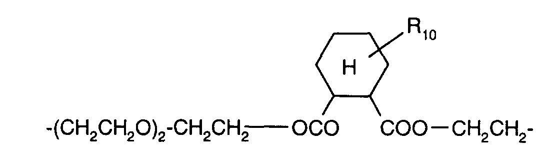 R₂ ist bevorzugt CH₃ oder R₁, wobei die gleichen Bevorzugungen, wie für R₁ gelten.

Die erfindungsgemässen Diketopyrrolopyrrole der Formel 1, worin R₁ einen Rest der Formel II oder III und R₂ C₁-C₆-Alkyl oder 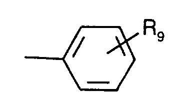 bedeuten, stellt man bevorzugt durch Umsetzung eines Pyrrolinons der Formel 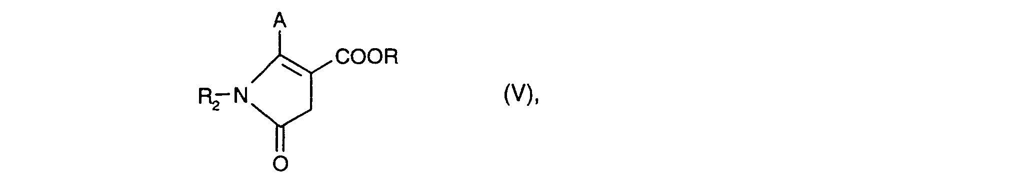 worin R bevorzugt C₁-C₄-Alkyl ist, mit einem Nitril der Formel

B-CN (VI)

her, wobei A und B die oben angegebene Bedeutung haben, unter Erhalt des Diketopyrrolopyrrols der Formel 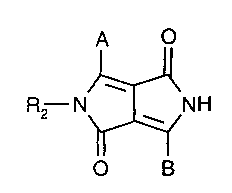 und weitere Umsetzung des letzteren mit einer Verbindung der Formel

R₁-Hal (VII)

oder, wenn Y im Rest mit der Formel III -CH₂-CH(OH)- bedeutet, mit einer Verbindung der Formel 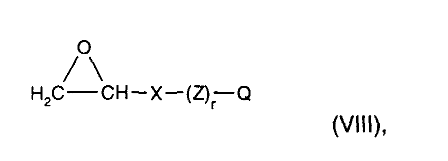 worin R₁, X, Z, Q und r die oben angegebene Bedeutung haben und Hal vorzugsweise Chlor oder Brom ist.

Die Wahl der Reaktionsparameter kann man beispielsweise analog zu dem in der US 4,778,899 beschriebenen Verfahren vornehmen, so daß sich nähere Angaben hierzu erübrigen.

Erfindungsgemässe Diketopyrrole der Formel 1, worin R₁ und R₂ gleich sind, erhält man in einer bevorzugten Ausführungsform in analoger Weise ausgehend von einem Diketopyrrolopyrrol der Formel 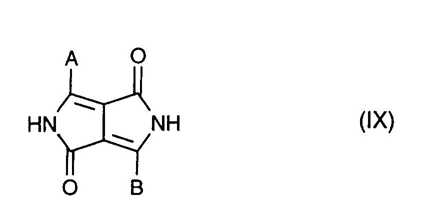 (z.B. erhältlich nach dem in der US 4,579,949 beschriebenen Verfahren) bei Verwendung entsprechender Mengen der Verbindungen der Formeln VII oder VIII.

Diketopyrrolopyrrole der Formel I, worin Q z.B. -CO-CH=CH₂ oder -CO-C(CH₃)=CH₂ und r 1 bedeuten, kann man in einer weiteren bevorzugten Ausführungsform durch Umsetzung von Diketopyrrolopyrrolen der Formel I, worin Q -OH ist, mit Acryl- bzw. Methacrylsäurechlorid hergestellt werden. In analoger Weise können z.B. auch die Gruppen -Si(Cl)₂-CH=CH₂, -Si(OC₂H₅)₂-CH=CH₂, -Si(OCOCH₃)₂-CH=CH₂, -CONHR₁₁, -COOR₁₁ usw. als Q bzw. Z-Q eingeführt werden.

Pyrrolinone der Formel V erhält man üblicherweise nach an sich bekannten Methoden, z.B. durch Cyclisierung einer Verbindung der Formel 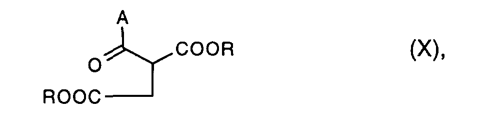 worin A und R die oben angegebene Bedeutung haben, mit einem Ammoniumsalz zum Pyrrolinon der Formel 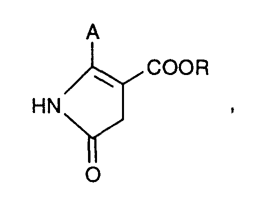 wie z.B. im US-Patent 4 778 899 beschrieben, und weitere Umsetzung mit einer Verbindung der Formel R₂-Hal, worin R₂ C₁-C₆-Alkyl oder 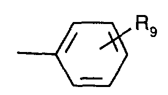 bedeutet, nach allgemein bekannten Methoden.

Die Verbindungen der Formeln VI, VII, VIII, IX und X sind bekannt und/oder können in Analogie zu allgemein bekannten Verfahren hergestellt werden.

Die erfindungsgemäßen DPP-Verbindungen kann man dank ihrer reaktiven Gruppen sehr gut zur Herstellung oder Modifizierung von Polymeren durch eine Polyreaktion oder eine polymeranaloge Reaktion verwenden. Die so hergestellten oder modifizierten farbigen Polymere weisen unerwartet interessante Farbeffekte und, je nach der Menge der eingesetzten DPP-Verbindung, verschiedenartigste Nuancen auf, die von jenen der entsprechenden DPP-Pigmente, die keine zu Polyreaktionen befähigten Gruppen besitzen, völlig abweichen können.

Des weiteren betrifft die vorliegende Erfindung ein Verfahren zur Herstellung oder Modifizierung von Polymeren durch Polyreaktion oder polymeranaloge Reaktion, indem man ein Diketopyrrolopyrrol der Formel I, gewünschtenfalls in Gegenwart eines geeigneten, bevorzugt üblichen, z.B. mindestens eine Kohlenstoff-Kohlenstoff-Doppelbindung tragenden Comonomeren oder eines polyreaktionsfähige Gruppen tragenden Polymeren, polymerisiert.

In einer bevorzugten Ausführungsform kann man gefärbte (Co-)Polymere herstellen, indem man eine Mischung aus erfindungsgemäßen DPP-Monomeren und weiteren, üblichen und geeigneten Monomeren in flüssiger Phase wie in Schmelze, Lösung, Suspension und Emulsion zur Polyreaktion bringt.

Die Herstellung dieser neuen DPP-Polymeren erfolgt üblicherweise nach allgemein bekannten Methoden, z.B. entweder durch eine Polyreaktion, d.h. durch Polymerisation (thermisch oder photochemisch), Polykondensation oder Polyaddition, oder durch eine polymeranaloge Reaktion, d.h. durch Umsetzung der erfindungsgemässen geeignete reaktive Gruppen enthaltenden DPP-Verbindungen mit bereits vorliegenden Polymeren, die selbst reaktionsfähige Gruppen aufweisen (Pfropfung).

Nach bisherigen Beobachtungen kann man mit den erfindungsgemäßen DPP-Verbindungen (DPP-Monomeren) alle bekannten Arten von Polyreaktionen durchführen. So können z.B. aus DPP-Monomeren, deren reaktive Gruppen C=C-Bindungen aufweisen, Vinyl-, Allyl-, Vinylester-, Vinylamid-, Vinylacetat- oder Vinylketon-Polymere, aus monofunktionellen DPP-Monomeren, deren reaktive Gruppen Heteroatome enthalten, Polyaldehyde, Polyisocyanate, Polyepoxide, Polyether, Polyacetone oder Polylactame, aus bifunktionellen DPP-Monomeren, deren reaktive Gruppen Heteroatome enthalten, via Polykondensation Polyester, Polyamide, Polyimide oder Polycarbonate und via Polyaddition, Polyepoxide, Polyurethane oder Polyimide hergestellt werden, wobei es sich bei der Polymerisation um radikalische, kationische oder anionische Polymerisation, Coordinationspolymerisation oder Gruppentransferpolymerisation handeln kann.

Beispiele für die Herstellung von DPP-Polymeren, ausgehend von den erfindungsgemässen DPP-Monomeren, sind:
Polymerisation: DPP-Polyacrylate durch radikalische thermische Polymerisation von DPP-Acrylaten; DPP-Polyacrylate durch radikalische Photopolymerisation von DPP-Acrylaten.
Polykondensation: DPP-Polyester aus DPP-Diolen und Disäurechloriden;
   DPP-Polycarbonate aus DPP-Diolen und Phosgen.
Polyaddition: DPP-Polyurethane aus DPP-Diolen und Diisocyanaten;
   DPP-Polyepoxide aus DPP-Epoxiden und Aminen
Polymeranaloge Reaktion: Umsetzung eines DPP-Alkohols mit einem aus Styrol und Maleinsäureanhydrid hergestellten und demnach Anhydridgruppen aufweisenden Polymer zu einem DPP-Mono- oder -diestergruppen enthaltenden Polymer.

Gegebenenfalls enthalten die neuen DPP-Polymeren auch Additive, wie Lichtschutzmittel, Antioxidantien und UV-Absorber, die während oder nach der eigentlichen Polymerisation zugegeben werden können, z.B. auch bei der Verarbeitung der Polymeren (Extrusion).

Diese Additive können auch selbst polyreaktionsfähige reaktive Gruppen aufweisen und in diesem Fall zusammen mit den DPP-Monomeren copolymerisiert werden.

Die erfindungsgemäß hergestellten DPP-Polymere, worunter im folgenden auch Copolymere, hergestellt aus erfindungsgemäßen DPP-Monomeren und anderen, üblichen Monomeren, zu verstehen sind, eignen sich vorteilhaft für viele Zwecke wie zur Einfärbung von hochmolekularen organischen Materialien wie Biopolymeren, Kunststoffen, inklusive Fasern, Gläsern, keramischen Produkten, für Zubereitungen in der dekorativen Kosmetik, zur Herstellung von Tinten, Druckfarben, Lacken, insbesondere Automobillacken und Photolacken, photo- und elektroleitenden Polymeren, fluoreszie-renden Aufhellern, Photozellen-Aggregaten, gefärbten Photoresists und Dispersionsfarben, des weiteren kann man die erfindungsgemäßen Diketopyrrolopyrrole im biomedizinischen Anwendungsbereich einsetzen wie zur Herstellung von Diagnostika sowie auf den Gebieten Impact- und Non-Impact-Printing und Photo/Repro allgemein.

Beispiele geeigneter hochmolekularer organischer Materialien, die mit den erfindungsgemäßen DPP-Polymeren gefärbt werden können, sind Vinylpolymere wie Polystyrol, Poly-α-methylstyrol, Poly-p-methylstyrol, Poly-p-hydroxystyrol, Poly-p-hydroxyphenylstyrol, Polymethylmethacrylat und Polyacrylamid sowie die entsprechenden Methacrylverbindungen, Polymethylmaleat, Polyacrylnitril, Polymethacrylnitril, Polyvinylchlorid, Polyvinyl-fluorid, Polyvinylidenchlorid, Polyvinylidenfluorid, Polyvinylacetat, Polymethylvinylether und Polybutylvinylether; von Maleinimid und/oder Maleinanhydrid abgeleitete Polymere wie Copolymere von Maleinanhydrid mit Styrol; Polyvinylpyrrolidon; ABS; ASA; Polyamide; Polyimide; Polyamidimide; Polysulfone; Polyethersulfone; Polyphenylenoxide; Polyurethane; Polyharnstoffe; Polycarbonate; Polyarylene; Polyarylensulfide; Polyepoxide; Polyolefine wie Polyethylen und Polypropylen; Polyalkadiene; Biopolymere und deren Derivate wie Cellulose, Celluloseether und -ester wie Ethylcellulose, Nitrocellulose, Celluloseacetat und Cellulosebutyrat, Stärke, Chitin, Chitosan, Gelatine, Zein; natürliche Harze; Kunstharze wie Alkydharze, Acrylharze, Phenolharze, Epoxidharze, Aminoformaldehydharze wie Harnstoff- und Melamin-Formaldehydharze; Gummi; Casein; Silikon und Silikonharze; Kautschuk, Chlorkautschuk; des weiteren Polymere, die beispielsweise als Bindemittel in Lacken verwendet werden wie Novolacke abgeleitet von C₁-C₆-Aldehyden wie Formaldehyd und Acetaldehyd und einem zweikernigen oder einkernigen, vorzugsweise einkernigen Phenol, das gewünschtenfalls mit einer oder zwei C₁-C₉-Alkylgruppen, einem oder zwei Halogenatomen oder einem Phenylring substituiert ist wie o-, m- oder p-Kresol, Xylol, p-tert.-Butylphenol, o-, m- oder p-Nonylphenol, p-Chlorphenol oder p-Phenylphenol, oder einer Verbindung mit mehr als einer phenolischen Gruppe wie Resorcin, Bis-(4-hydroxyphenyl)methan oder 2,2-Bis-(4-hydroxyphenyl)propan; sowie geeignete Mischungen der genannten Materialien.

Besonders bevorzugte hochmolekulare organische Materialien, insbesondere zur Herstellung eines Lackes, einer Druckfarbe oder Tinte, sind beispielsweise Celluloseether und -ester, wie Ethylcellulose, Nitrocellulose, Celluloseacetat und Cellulosebutyrat, natürliche Harze oder Kunstharze (Polymerisations- oder Kondensationsharze) wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, ASA, Polyphenylenoxide, Gummi, Casein, Silikon und Silikonharze sowie deren mögliche Mischungen untereinander.

Man kann auch hochmolekulare organische Materialien in gelöster Form als Filmbildner einsetzen wie Leinölfirnis, Nitrocellulose, Alkydharze, Phenolharze, Melamin- und Harnstoff/Formaldehydharze sowie Acrylharze.

Die genannten hochmolekularen organischen Verbindungen können einzeln oder in Gemischen z.B. als Granulat, plastische Massen, Schmelzen oder in Form von Lösungen, insbesondere zur Herstellung von Spinnlösungen, Lacken, Anstrichstoffen, Tinten oder Druckfarben, vorliegen.

In einer besonders bevorzugten Ausführungsform verwendet man die erfindungsgemäßen DPP-Polymere zum Massefärben von Polyvinylchlorid, Polyamiden und insbesondere Polyolefinen wie Polyethylen und Polypropylen sowie zur Herstellung von Lacken, inklusive Pulverlacken, Tinten, Druckfarben und Anstrichfarben.

Als Beispiele für bevorzugte Bindemittel für Lacksysteme seien Alkyd/Melaminharzlacke, Acryl/Melaminharzlacke, Celluloseacetat/Cellulosebutyratlacke und Zweikomponentenlacke auf Basis mit Polyisocyanat vernetzbarer Acrylharze genannt.

Nach bisherigen Beobachtungen kann man die erfindungsgemäßen DPP-Polymere in jeder gewünschten Menge in Abhängigkeit vom Verwendungszweck dem zu färbenden Material zugeben. Beispielsweise kann man bei hochmolekularen organischen Materialien die erfindungsgemäßen zusammengesetzten Pigmente in einer Menge im Bereich von 0,01 bis 40, bevorzugt von 0,1 bis 20 Gew.-%, bezogen auf das Gesamtgewicht des gefärbten hochmolekularen organischen Materials, einsetzen.

Die Einfärbung der hochmolekularen organischen Materialien mit den erfindungsgemäßen DPP-Polymeren erfolgt in der Regel dergestalt, daß man die erfindungsgemäßen DPP-Polymere, gewünschtenfalls in Form von Masterbatches, den hochmolekularen organischen Materialien unter Verwendung üblicher hierzu geeigneter Vorrichtungen wie Extruder, Walzwerke, Misch- oder Mahlapparaturen zumischt. Das so behandelte Material wird im allgemeinen hierauf nach an sich bekannten Verfahren wie Kalandrieren, Pressen, Strangpressen, Streichen, Gießen, Extrudieren oder Spritzgießen in die gewünschte endgültige Form gebracht.

In einer bevorzugten Ausführungsform kann man die erfindungsgemäßen DPP-Monomeren zusammen mit anderen Monomeren, insbesondere solchen, die üblicherweise zur Herstellung der bereits weiter oben genannten Polymere eingesetzt werden, in einem Extruder zur Polyreaktion bringen (reactive extrusion, analog zu beispielsweise dem in der EP-A 337 951 beschriebenen Verfahren). So hergestellte Copolymere weisen üblicherweise die gleiche-Anwendungsbreite auf wie die bislang genannten Blends aus erfindungsgemäßen DPP-Polymeren und hochmolekularen organischen Materialien.

Zur Herstellung nicht starrer Formlinge oder zur Verringerung ihrer Sprödigkeit kann man den hochmolekularen Substanzen vor der Verformung sogenannte Weichmacher zusetzen. Weichmacher können beispielsweise sein: Ester der Phosphorsäure, Phthalsäure und Sebazinsäure. Die Weichmacher können vor, während oder nach dem Einfärben der hochmolekularen Substanzen mit den erfindungsgemäßen DPP-Polymeren zugesetzt werden.

Zur Erzielung verschiedener Farbtöne kann man die erfindungsgemäßen DPP-Polymere vorteilhaft in Abmischung mit Füllstoffen, transparenten und deckenden Weiß-, Bunt- und/oder Schwarzpigmenten sowie auch herkömmlichen Glanzpigmenten in der gewünschten Menge einsetzen.

Zur Herstellung von Lacken, Anstrichstoffen, Tinten und Druckfarben dispergiert oder löst man im allgemeinen die entsprechenden hochmolekularen organischen Substanzen wie Bindemittel, Kunstharzdispersionen etc. und die erfindungsgemäßen DPP-Polymere, gewünschtenfalls zusammen mit üblichen Zusatzstoffen wie Füllmitteln, Lackhilfsmitteln, Siccativen, Weichmachern und/oder zusätzlichen Pigmenten, in einem gemeinsamen Lösungsmittel oder Lösungsmittelgemisch. Man kann dabei so verfahren, daß man die einzelnen Komponenten für sich oder auch mehrere gemeinsam dispergiert oder löst, und erst hierauf alle Komponenten zusammenbringt, oder alles auf einmal zugibt.

Bei der Applikation im Druck kann man alle industrieüblichen Druckverfahren wie Siebdruck, Tiefdruck, Bronzierdruck, Flexodruck und Offestdruck einsetzen.

Die folgenden Beispiele erläutern die Erfindung.

### Herstellung von DPP-Monomeren

Beispiel 1a): 29,8 g (0,12 Mol) des Pyrrolinons der Formel 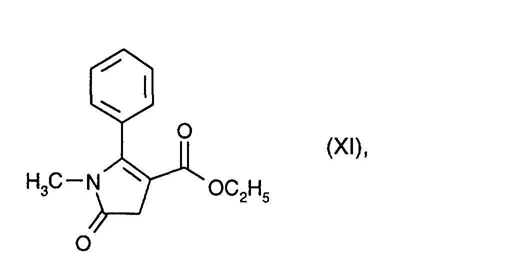 hergestellt in Anlehnung an die in Beispiel 4 von US-Patent 4,778,899 beschriebenen Methode, und 18,16 g (0,132 Mol) p-Chlorbenzonitril werden unter Stickstoffspülung in einem Sulfierkolben vorgelegt und noch 180 ml 2-Methyl-1-pentanol dazugegeben. Das Gemisch wird auf 110-115°C erhitzt bis, nach etwa 30 Minuten, eine klare hellbraune Lösung entsteht. Danach werden innert 2 Stunden 12,96 g (0,24 Mol) 30%iges Natriummethylat in gesättigter Natronlauge bei einer Rührgeschwindigkeit von 510-520 U/Minute und gleich zeitigem Abdestillieren des sich bildenden Methanol/Ethanol-Gemisches zudosiert. Es wird etwa eine Stunde bei derselben Temperatur weitergerührt und dann auf Zimmertemperatur abgekühlt. Nach Zugabe von 500 ml Methanol wird mit 57,7 g (0,96 Mol) Essigsäure angesäuert. Nach kräftigem Rühren des sich bildenden dicken Breis fällt nach etwa 3 Minuten das Produkt in intensiv orangeroter Farbe aus. Man verdünnt mit weiteren 100 ml Methanol und 150 ml Wasser und filtriert die orangerote Suspension. Der Filterkuchen wird mit 150 ml Methanol in 3 Portionen gewaschen und über Nacht im Vakuumtrockenschrank bei 60-70°C getrocknet. Man erhält 25,53 g (63,2 % d.Th.) des Diketopyrrolopyrrols der Formel

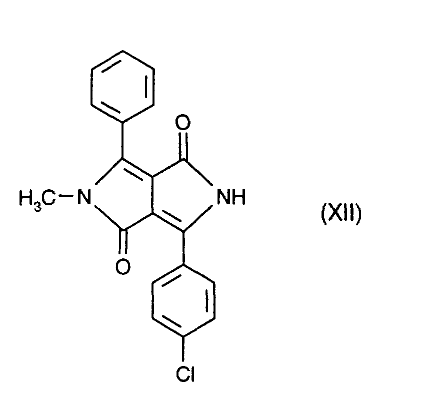

| Analyse | C | H | N | Cl |
|---|---|---|---|---|
| Berechnet | 67,76 % | 3,89 % | 8,32 % | 10,53 % |
| Gefunden | 67,62 % | 3,93 % | 8,26 % | 10,47 % |

b) 10,10 g (0,030 Mol) des Diketopyrrolopyrrols der Formel XII (Beispiel 1a), 2,50 g (0,018 Mol) Kaliumcarbonat und 100 ml Dimethylacetamid werden unter Stickstoffspülung in einem Sulfierkolben vorgelegt und das Gemisch wird unter Rühren auf 130-135°C erhitzt. Man erhält nach 30 Minuten bei dieser Temperatur eine stark dunkelrotbraune Suspension, in welche innert 1 Stunde 7,23 g (0,0375 Mol) 10-Chlor-1-decanol mit 10 ml Dimethylacetamid verdünnt zugetropft werden. Nach 4 Stunden Rühren bei der gleichen Temperatur werden weitere 2,90 g (0,015 Mol) 10-Chlor-1-decanol mit 10 ml Dimethylacetamid verdünnt innert 15 Minuten zugetropft und noch weitere 4 Stunden stets unter Stickstoffspülung gerührt, bevor auf Raumtemperatur abgekühlt wird. Danach wird die dunkle rotbraune Lösung kalt filtriert, der Filterrückstand wird dreimal mit 5 ml Dimethylacetamid gewaschen und das Filtrat im Rotationsverdampfer vollständig eingeengt. Das Rohprodukt wird mittels Säulenchromatographie über Kieselgel 60 mit Toluol/Dioxan 9:1 als Eluiermittel gereinigt. Man erhält 6,25 g (42,3%) der Verbindung der Formel 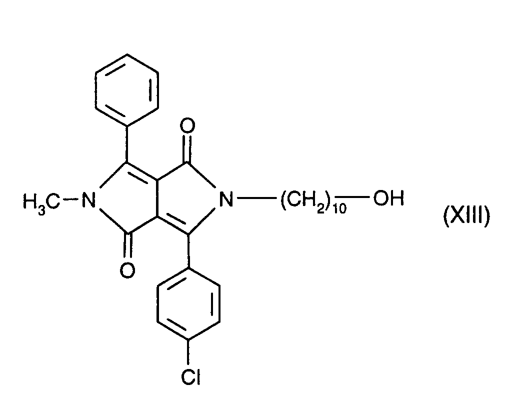 als dunkelrotes Öl.

Das NMR-Spectrum in CDCl₃/p.a. stimmt mit der Zielstruktur voll überein.

Beispiel 2: In einem gut mit Stickstoff gespülten Sulfierkolben werden 26,94 g (0,08 Mol) des Diketopyrrolopyrrols der Formel XII (Beispiel 1a) und 26,85 g (0,195 Mol) Kaliumcarbonat (beide Substanzen vorgängig bei 350°C getrocknet) eingewogen und 350 ml Dimethylformamid dazugegeben. Das Gemisch wird unter Rühren auf 120-125°C erhitzt und dann 50,50 g (0,195 Mol) 97%iges 11-Brom-1-undecanol in 100 ml Dimethylformamid gelöst innert 15 Minuten bei dieser Temperatur zugetropft. Nach beendeter Zugabe liegt eine dunkelrotbraune Suspension vor, die bei 120-125°C 2 Stunden weitergerührt und dann auf Raumtemperatur abgekühlt wird, wobei die Farbe auf Gelbbraun umschlägt. Es wird noch über Nacht bei Raumtemperatur weiter gerührt, anschliessend filtriert und der Filterrückstand mit 30 ml Dimethylformamid nachgespült. Das Filtrat wird im Rotationsverdampfer vollständig eingeengt. Das Rohprodukt wird mittels Säulenchromatographie über Kieselgel 60 mit Toluol/Dioxan 8:2 als Eluiermittel gereinigt. Man erhält 9,4 g (23,2% d.Th.) der Verbindung der Formel

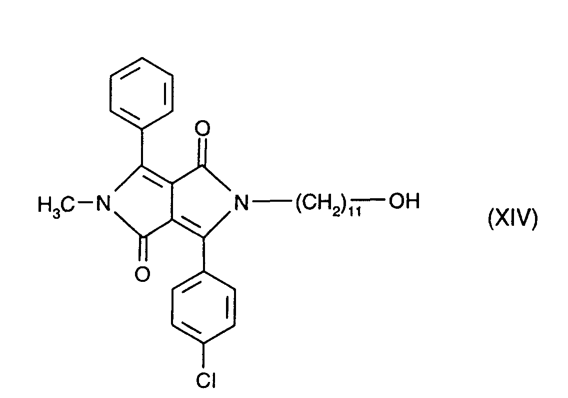

Das NMR-Spectrum in CDCl₃/p.a. stimmt mit der Zielstruktur überein.

Beispiel 3: In einem Sulfierkolben werden unter Stickstoff 6,73 g (0,012 Mol) des Diketopyrrolopyrrols der Formel XIV (Beispiel 2), 0,012 g (0,06 mMol) Phenothiazin und 110 ml Dichlorethan vorgelegt. Nach dem Aufheizen bis zur Rückflusstemperatur der entstandenen gelbroten Lösung werden 2,17 g (0,024 Mol) in 10 ml Dichlorethan gelöstes Acrylsäurechlorid innert 15 Minuten zugetropft. Nach beendeter Zugabe wird 7 Stunden am Rückfluss (80°C) weitergerührt und danach auf Raumtemperatur abgekühlt. Die orangerote Lösung wird im Scheidetrichter 5 Mal mit 30 ml 5%iger Natronlauge und anschliessend 2 Mal mit deionisiertem Wasser ausgeschüttelt. Die organische Phase wird mit MgSO₄ • H₂O getrocknet und filtriert. Nach vollständigem Einengen des orangeroten Filtrats im Rotationsverdampfer wird das Rohprodukt aus 60 ml Methanol umkristallisiert. Man erhält 6,1 g (81,9% d.Th.) eines orangefarbenen Produkts der Formel

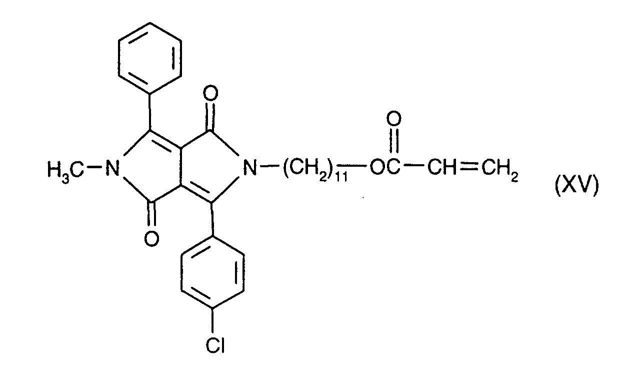

| Analyse | C | H | N | Cl |
|---|---|---|---|---|
| Berechnet | 70,64 % | 6,5 % | 4,99 % | 6,32 % |
| Gefunden | 70,30 % | 6,81 % | 5,10 % | 6,38 % |

Beispiel 4a): In einem Sulfierkolben werden unter Stickstoff 29,43 g (0,12 Mol) des Pyrrolinons der Formel Xl (Beispiel 1a) und 21,02 g (0,132 Mol) 4-tert.-Butyl-benzonitril vorgelegt und 180 ml 2-Methyl-1-pentanol zugegeben. Dann wird unter Rühren auf 115-120°C erhitzt bis, nach etwa 30 Minuten eine klare hellbraune Lösung entsteht. Danach werden innert 2 Stunden 43,22 g (0,24 Mol) 30%iges Natriummethylat in gesättigter Natronlauge bei einer Rührgeschwindigkeit von 510-520 U/Minute wund gleichzeitigem Abdestillieren des sich bildenden Methanol/Ethanol-Gemisches zudosiert. Nach beendeter Zugabe des Natriummethylats entsteht eine dunkelviolette Lösung, die 30 Minuten bei 115-120°C weitergerührt und anschliessend auf Raumtemperatur abgekühlt wird. Nach Zugabe von 600 ml Methanol wird mit 57,7 g Essigsäure angesäuert und mit 600 ml Wasser verdünnt. In der grüngelben Lösung bildet sich ein dunkles Öl, aus dem durch Verrühren über Nacht ein oranges Pigment auskristallisiert. Dieses wird abfiltriert, mehrmals mit Methanol gewaschen und bei 40-50°C im Vakuumtrockenschrank getrocknet. Man erhält 3,84 g eines rotorangen kristallinen Produktes der Formel

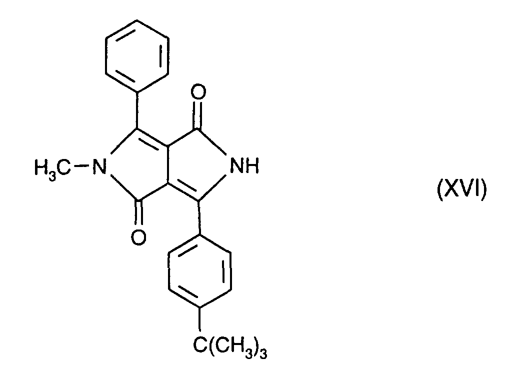

b) Verfährt man wie in Beispiel 1b beschrieben, ersetzt man aber das Diketopyrrolopyrrol der Formel XII durch die äquivalente Menge des Diketopyrrolopyrrols der Formel XVI, so erhält man die Verbindung der Formel

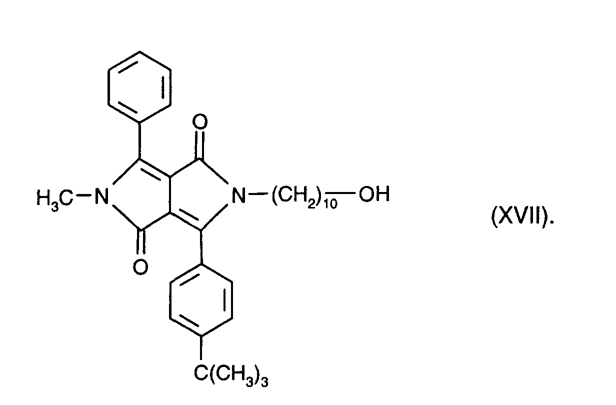

Beispiel 5: Verfährt man wie in Beispiel 1b beschrieben, ersetzt man aber das 10-Chlor-1-decanol durch die äquivalente Menge 6-Chlor-1-hexanol, so erhält man die Verbindung der Formel

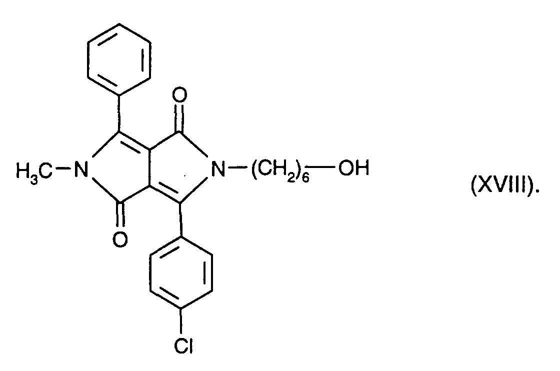

Beispiel 6: 20,21 g (0,060 Mol) des Diketopyrrolopyrrols der Formel XII (Beispiel 1a) werden in einem gut mit Stickstoff gespülten Sulfierkolben direkt eingewogen, dann werden 270 ml Dimethylformamid dazugegeben und unter Rühren und Stickstoffzufuhr auf 130-135°C erhitzt. Nach 30 Minuten werden 12,44 g (0,090 Mol) Kaliumcarbonat (bei 250°C getrocknet) zugegeben. In die dunkelviolette Suspension werden 15,18 g (0,090 Mol) in 30 ml Dimethylformamid gelöstes Triethylenglykolmonohydrin innert 15 Minuten zugetropft. Die entstandene dunkelbraune Suspension wird 4½ Stunden bei 130-135°C gerührt, dann auf Raumtemperatur abgekühlt und noch über Nacht weitergerührt. Danach wird filtriert und der Filterrückstand wird mit Dimethylformamid gewaschen. Das Filtrat wird im Rotationsverdampfer bis auf etwa 150 ml eingeengt. Das Produkt kristallisiert aus und wird aus 200 ml Ethanol umkristallisiert und bei 40-50°C im Vakuumtrockenschrank getrocknet. Man erhält 8,42 g (30% d.Th.) eines kristallinen Produktes der Formel 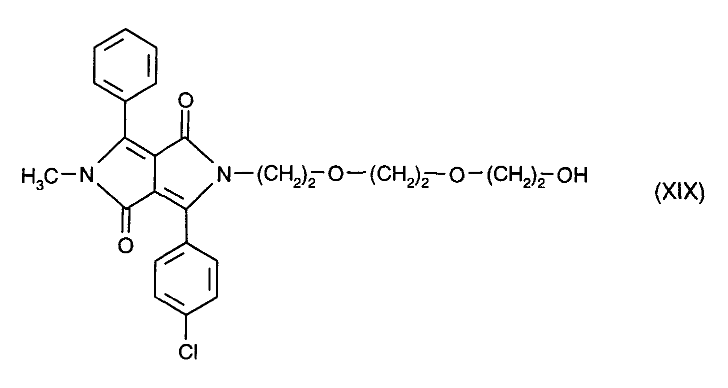 mit Schmelzpunkt 163°C.

Beispiele 7-9: Setzt man in analoger Weise, wie in Beispiel 3 beschrieben, ein Diketopyrrolopyrrol der Formel 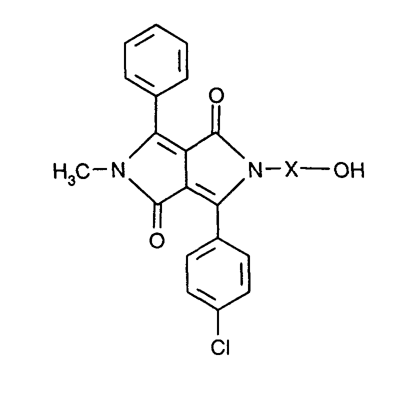 mit einem Säurechlorid der Formel 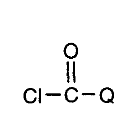 um, so erhält man ein Produkt der Formel 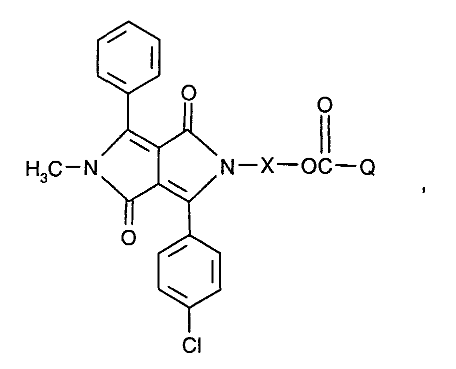 wobei X und Q die in der nachstehenden Tabelle angegebene Bedeutung haben

| Beispiel | X | Q |
|---|---|---|
| 7 | -(CH₂)₆- | -C(CH₃)=CH₂ |
| 8 | -(CH₂)₁₀- | -CH=CH₂ |
| 9 | -(CH₂)₂-O-(CH₂)₂-O-(CH₂)₂- | -CH=CH₂ |

Beispiel 10: 2,35 g (0,005 Mol) des Diketopyrrolopyrrols der Formel XIX (Beispiel 6) und 30 ml Dichlorbenzol werden unter Stickstoff in einem Sulfierkolben vorgelegt, dann werden 5,0 mg (0,05 mMol) Thiodiphenylamin zugegeben und unter Rühren auf 110-115°C erhitzt. In die klare orangerote Lösung werden 3,17 g (0,010 Mol) des Methyl-Hexahydrophthalsäurederivats (Isomerengemisch) der Formel 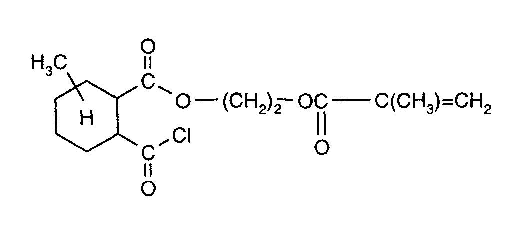 (nach allgemein bekannten Methoden aus Methylhexahydrophthalsäureanhydrid und Hydroxyethylmethacrylat erhalten) unter kräftigem Rühren innert 15 Minuten zugetropft, etwa 2 Stunden bei 110-115°C weitergerührt und dann auf Raumtemperatur abgekühlt. Die dunkelrote klare Lösung wird im Scheidetrichter 3 Mal mit 20 ml 5 %iger Natronlauge und 2 Mal mit 30 ml deionisertem Wasser gewaschen, die organische Phase mit MgSO₄ • H₂O getrocknet, filtriert und im Hochvakuum vollständig eingeengt. Das erhaltene Produkt wird aus 30 ml Ethanol umkristallisiert. Man erhält 3,05 g (81,3% d.Th.) eines kristallinen Produktes der Formel 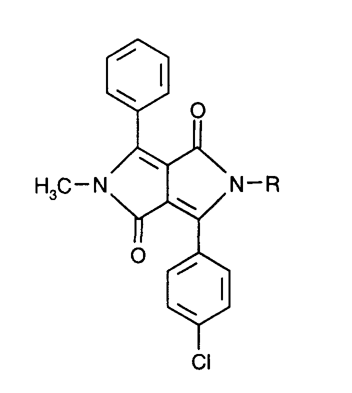 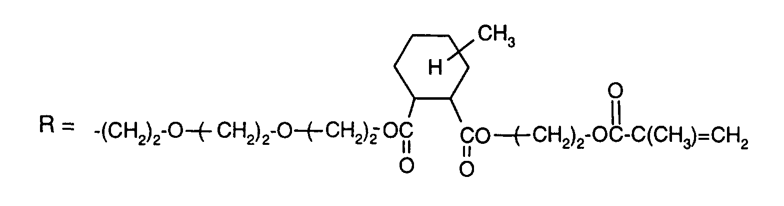 mit Schmelzpunkt 69,6°C.

Beispiel 11: 34,60 g (0,12 Mol) Diketopyrrolopyrrol der Formel 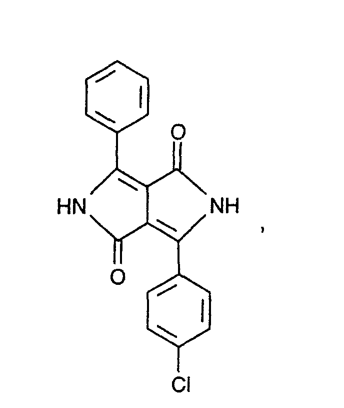 (erhalten nach der in US 4,579,949 (Bsp. 26) beschriebenen Methode) 49,76 g (0,36 Mol) Kaliumcarbonat (bei 350°C getrocknet) und 600 ml frisch destilliertes Dimethylformamid werden unter Stickstoff in einem Sulfierkolben vorgelegt und auf 130-135°C erhitzt. Dann werden 51,80 g (0,36 Mol) 95%iges 6-Chlor-1-hexanol unter kräftigem Rühren innert etwa 10 Minuten bei dieser Temperatur zugetropft. Nach beendeter Zugabe wird die dunkelrote Suspension noch weitere 2 Stunden bei 130-135°C und dann bei Raumtemperatur über Nacht weitergerührt. Die entstandene dunkelrote Lösung mit suspendiertem Material (KCI, K₂CO₃) wird filtriert und der Filterkuchen wird dreimal mit 25 ml Dimethylformamid gewaschen. Das Filtrat wird unter gutem Rühren zu 2 l destilliertem Wasser gegeben, wobei ein orangefarbenes Produkt ausfällt. Das Gemisch wird zum Sieden erhitzt und bei 94°C filtriert. Die im Filter zurückgebliebene klebrige rote Masse wird in 800 ml Ethanol heissgelöst, auf ca 300 ml eingeengt und über Nacht stehen gelassen, wobei ein orangefarbiges Produkt ausfällt, das nach dem Abkühlen in Eiswasser abfiltriert und in Ethanol umkristallisiert wird. Man erhält 15,1 g (26,5% d.Th.) eines orangen kristallinen Produkt der Formel

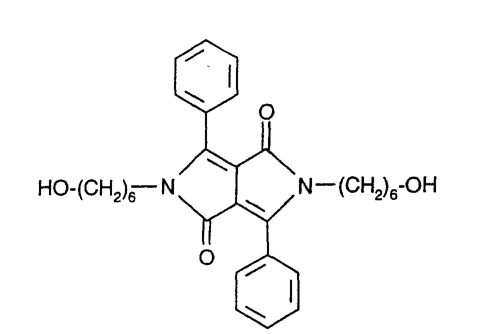

| Analyse | C | H | N |
|---|---|---|---|
| Berechnet | 73,74 % | 7,43 % | 5,73 % |
| Gefunden | 73,51 % | 7,47 % | 5,66 % |

Beispiel 12: 19,55 g (0,04 Mol) des Produktes von Beispiel 11, 0,04 g (2•10⁻⁴ Mol) Phenothiazin und 380 ml Dichlorethan werden unter Stickstoff in einem Sulfierkolben vorgelegt und zum Rückfluss geheizt. Der trüben orangefarbenen Lösung werden innert etwa 30 Minuten unter Rückfluss (80°C) und Rühren 14,48 g (0,16 Mol) Acrylsäurechlorid zugetropft. Nach beendeter Zugabe wird mit 20 ml Dichlorethan nachgespült. Die gelborange Lösung wird 7 Stunden am Rückfluss gerührt, anschliessend auf Raumtemperatur und über Nacht stehen gelassen. Die orangerote, ganz leicht trübe Lösung wird im Scheidetrichter 5 Mal mit 100 ml 5%iger Natronlauge und 2 Mal mit deionisiertem Wasser gewaschen und mit MgSO₄ • H₂O getrocknet und filtriert. Das orangerote klare Filtrat wird im Rotationsverdampfer vollständig eingeengt. Das zurückgebliebene rote Öl erstarrt über Nacht zu einer festen Masse, die aus Ethanol umkristallisiert wird. Man erhält 23,1 g (96,7% d.Th.) eines kristallinen Produktes der Formel 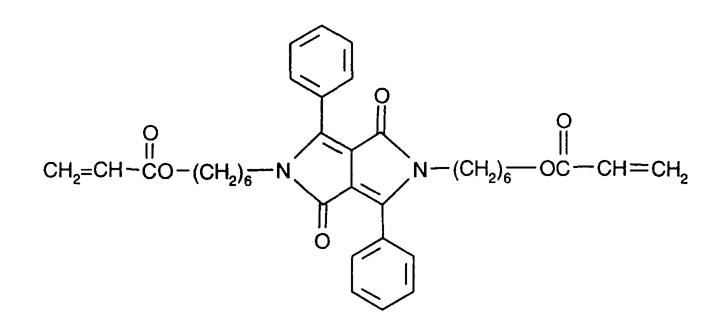 mit Schmelzpunkt 79,7-80,1°C.

### Herstellung von DPP-Polymeren

Beispiel 13: 5,13 g (0,01 Mol) des Produktes von Beispiel 11 und 60 ml Chlorbenzol werden unter Stickstoff in einem Sulfierkolben vorgelegt und zum Rückfluss geheizt. Bei 120°C werden weitere 30 ml Chlorbenzol zugegeben. In die leicht trübe Lösung werden bei 130°C unter Rühren 1,68 g (0,01 Mol) Hexamethylendiisocyanat innert 15 Minuten zugetropft. Etwa 40 Minuten nach beendeter Zugabe bei unveränderter Temperatur liegt eine klare Lösung vor. Nach 75 Minuten Reaktionszeit werden 0,013 g Dimethylcyclohexylamin (1,0 mol-%ige Lösung in Chlorbenzol) als Katalysator zugegeben. Nach weiteren 3½ Stunden geht die Lösung in eine feine Suspension über. Es wird über Nacht bei 129°C weitergerührt und anschliessend auf Raumtemperatur abgekühlt. die orangerote Suspension wird filtriert und das Produkt bei 60-70°C im Vakuum getrocknet. Man erhält 6,3 g (92,5 % d.Th.) des gewünschten Polyurethans mit einem scharfem Schmelzpunkt von 142,8°C. Im IR (KBr Pressling) kann die charakteristische Urethan-Bande bei 2330 cm⁻¹ klar erkannt werden.

### Beispiel 14: Photohärtung eines DPP-Bisacrylat-Monomers

0,8 g des Produktes von Beispiel 12 werden mit 7,2 g Cibatool®SL 5154 (eine Mischung, enthaltend Acrylat-Monomere, Photoinitiator und Sensibilisator, Fa. CIBA-GEIGY AG) bei 60°C vermischt und die erhaltene orangerote Lösung wird im Vakuum entgast.

Mit einem Erichsen Ziehdreieck werden ca. 100 µm-dicke Filme auf Glas gezogen, die anschliessend mit Hilfe einer Hoenle-UV-Lampe aus einer Distanz von 20 cm und Einstellung auf 60 % belichtet werden.

| Belichtungszeit (Min.) | Beurteilung |
|---|---|
| 1 | Der Film ist noch flüssig |
| 2 | orange-gelber Film, noch weich |
| 3 | orange-gelber fester Film. z.T. DMF*-löslich |
| 5 | dto. |
| 10 | orange-gelber fester Film geringe DMF**-Löslichkeit |
| 15 | orange-gelber fester Film nicht DMF* -löslich ! |
| 30 | dto. |

| | |
|---|---|
| * DMF = Dimethylformamid | |

Die Zusammenstellung zeigt anhand der abnehmenden Löslichkeit deutlich, dass das DPP-Bisacrylat, in dieser Formulierung, nach 15 Minuten vollständig polymerisiert ist.

## Patentansprüche

1. 1,4-Diketopyrrolopyrrole der Formel 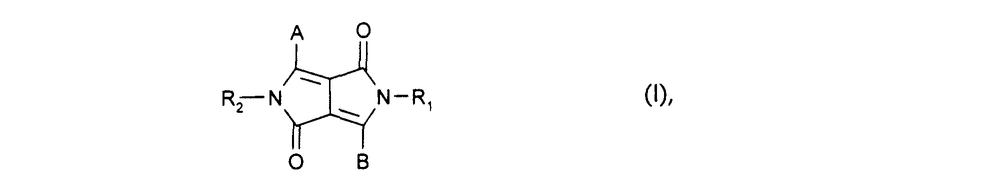 worin A und B unabhängig voneinander für eine Gruppe der Formel 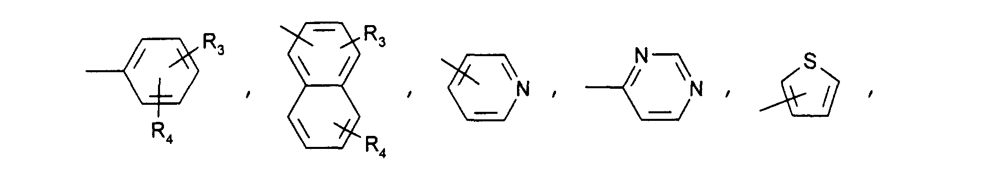 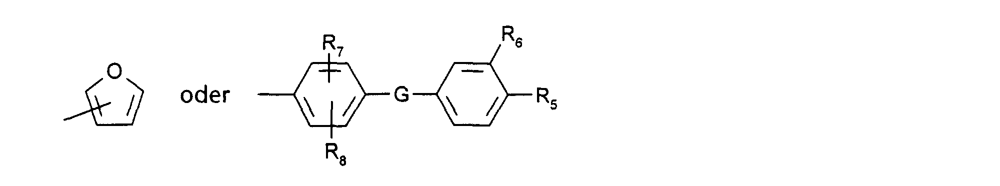 stehen, worin
R₃ und R₄ unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, C₁-C₁₈-Alkoxycarbonyl, C₁-C₁₈-Alkylaminocarbonyl, -CN, -NO₂, Trifluormethyl, C₅-C₆-Cycloalkyl, -C=N-(C₁-C₁₈-Alkyl), 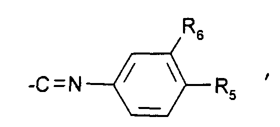 Imidazolyl, Pyrrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,
G -CH₂-, -CH(CH₃)-, -C(CH₃)₂-, -CH=N-, -N=N-, -O-, -S-, -SO-, -SO₂-, -CONHoder -NR₉- ist,
R₅ und R₆ unabhängig voneinander Wasserstoff, Halogen, C₁-C₆-Alkyl, C₁-C₁₈₋ Alkoxy oder -CN sind, R₇ und R₈ unabhängig voneinander Wasserstoff, Halogen oder C₁-C₆-Alkyl und R₉ Wasserstoff oder C₁-C₆-Alkyl bedeuten,
R₁ einen Rest der Formel
-(CH₂)ₘ-CH=CH-(CH₂)ₙ-CH₃ (II),
oder
-(Y)ₚ-X-(Z)ᵣ-Q (III)
bedeutet, worin m und n unabhängig voneinander eine ganze Zahl zwischen Null und 12 sind, mit der Bedingung, dass die Summe m + n mindestens 4 ist, und p und r unabhängig voneinander Null oder 1 bedeuten, mit der Massgabe, dass r nicht Null ist, falls p für Null und Q für -OH oder -COOR₁₁ stehen,
X ununterbrochenes oder ein- oder mehrfach durch -O- und/oder -S-, -NH-, Phenylen, -COO-, -CONH-, 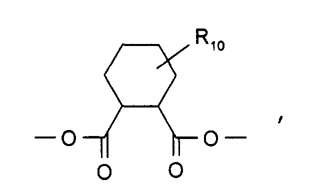 worin R₁₀ Wasserstoff oder Methyl ist, unterbrochenes C₄-C₁₈-Alkylen ist, 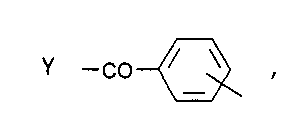 -Si(Cl)₂-, -Si(OC₂H₅)₂-, -Si(OCOCH₃)₂-, -CH₂-CH(OH)- oder -CH(CN)- und
Z -O-, -NH-, -COO-, Phenylen, 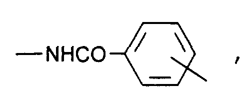 -Si(Cl)₂-, -Si(OC₂H₅)₂-, -Si(OCOCH₃)₂-
bedeuten,
Q -OH, -NH₂, Glycidyl, 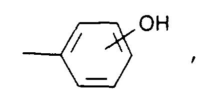 -CHO, -NCO, -CH=CH₂, C(CH)=CH₂, -CO-CH=CH₂, -CO-C(CH₃)=CH₂, C₅-C₇-Cycloalkenyl, 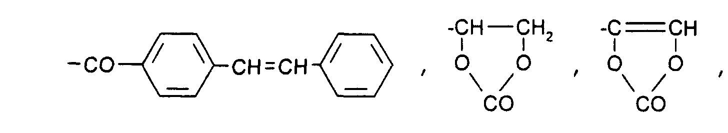 -CONHR₁₁, -COOR₁₁, -COR₁₁, worin R₁₁ Wasserstoff oder C₁-C₆-Alkyl ist, 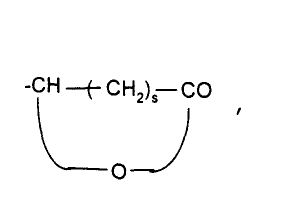 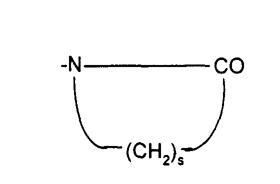 wobei s eine ganze Zahl von 1 bis 6 ist, bedeutet, und
R₂ C₁-C₆-Alkyl, 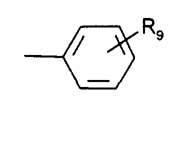 oder R₁ ist.

2. Diketopyrrolopyrrole gemäss Anspruch 1, **dadurch gekennzeichnet, dass** in der Formel I A und B unabhängig voneinander eine Gruppe der Formel 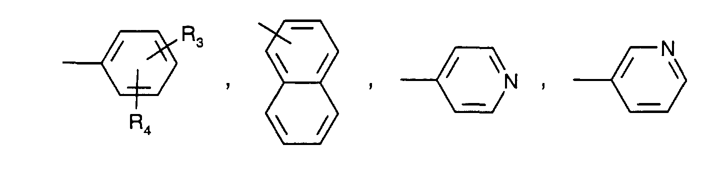 oder 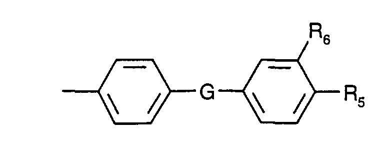 sind, worin R₃ und R₄ unabhängig voneinander Wasserstoff, Halogen, insbesondere Chlor oder Brom, C₁-C₄-Alkyl, C₁-C₆-Alkoxy, C₁-C₆-Alkylamino oder CN bedeuten,
G -O-, -NR₉-, -N=N- oder -SO₂- ist,
R₅ und R₆ Wasserstoff R₉ Wasserstoff, Methyl oder Ethyl bedeuten.

3. Diketopyrrolopyrrole gemäss Anspruch 2, **dadurch gekennzeichnet, dass** in der Formel I A und B gleich sind und eine Gruppe der Formel 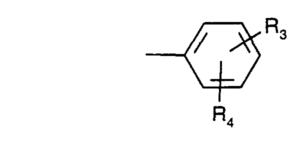 bedeuten, worin R₃ und R₄ unabhängig voneinander Wasserstoff, Methyl, tert.-Butyl, Chlor, Brom oder CN sind.

4. Diketopyrrolopyrrole gemäss Anspruch 1, **dadurch gekennzeichnet, dass** in der Formel I
R₁ einen Rest der Formel
-X-(O)ᵣ-Q (IV)
bedeutet, worin
X ununterbrochenes oder 1, 2 oder 3 Mal durch O und/oder einmal durch -S-, -NH-, 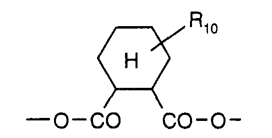 unterbrochenes C₄-C₁₂-Alkylen ist,
r und R₁₀ die in Anspruch 1 angegebene Bedeutung haben, und
Q -OH; -CH=CH₂, -C(CH₃)=CH₂, -CO-CH=CH₂ oder -CO-C(CH₃)=CH₂ bedeutet.

5. Diketopyrrolopyrrole gemäss Anspruch 4, **dadurch gekennzeichnet, dass** X vorzugsweise -(CH₂)_{q}-, wobei q eine ganze Zahl zwischen 6 und 12 sein kann,
-(CH₂CH₂O)₂-CH₂CH₂-
oder 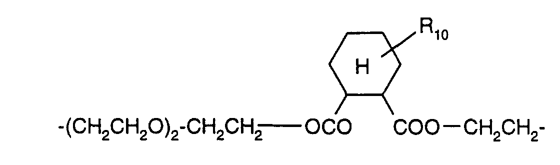 bedeutet.

6. Verfahren zur Herstellung von Diketopyrrolopyrrolen gemäss den Ansprüchen 1 bis 5 durch Umsetzung eines Pyrrolinons mit einem Nitril, **dadurch gekennzeichnet, daß** man (a) ein Pyrrolinon der Formel 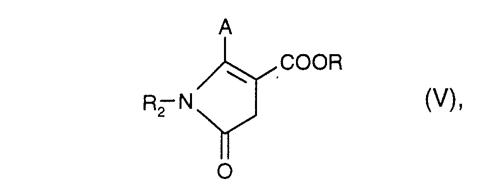 worin R bevorzugt C₁-C₄-Alkyl ist, mit einem Nitril der Formel
B-CN (Vi)
umsetzt, wobei A und B die in Anspruch 1 angegebene Bedeutung haben, R₁ für einen Rest der Formel II oder III wie in Anspruch 2 definiert und R₂ für C₁-C₆-Alkyl oder 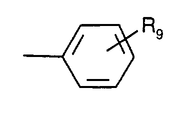 wie in Anspruch 1 definiert stehen, und (a1) das entstandene Diketopyrrolopyrrol mit einer Verbindung der Formel
R₁-Hal (VII)
oder, wenn Y im Rest mit der Formel III wie in Anspruch 2 definiert -CH₂-CH(OH)- bedeutet, mit einer Verbindung der Formel 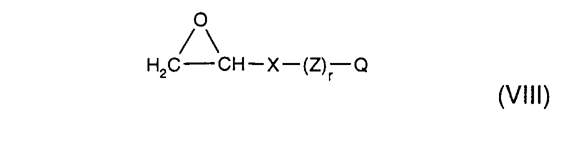 umsetzt, worin R₁, X, Z, Q und r die in Anspruch 2 angegebene Bedeutung haben und Hal vorzugsweise Chlor oder Brom ist, oder (a2) ein Diketopyrrolopyrrol der Formel 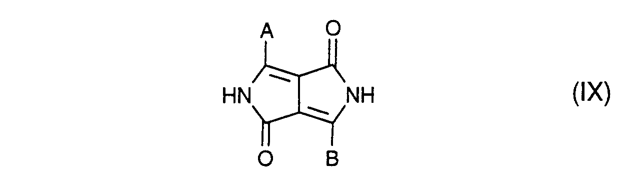 mit den Verbindungen der Formeln VII oder VIII in entsprechender Weise wie in (a1) umsetzt, oder
(b) Diketopyrrolopyrrole der Formel I, in denen R₁ einen Rest der Formel III wie in Anspruch 2 definiert mit Q = -OH ist, mit Acryl- oder Methacrylsäurechlorid umsetzt.

7. Verfahren zur Herstellung oder Modifizierung von Polymeren durch Polyreaktion oder polymeranaloge Reaktion, **dadurch gekennzeichnet, daß** man ein Diketopyrrolopyrrol der Formel I, gewünschtenfalls in Gegenwart eines geeigneten Comonomeren oder eines polyreaktionsfähige Gruppen tragenden Polymeren, polymerisiert.

8. Verwendung von Diketopyrrolopyrrolen gemäss den Ansprüchen 1 bis 5 oder hergestellt gemäss Anspruch 6 zur Herstellung oder Modifizierung von Polymeren durch eine Polyreaktion oder eine polymeranaloge Reaktion.

9. Mit Diketopyrrolopyrrolen gemäss den Ansprüchen 1 bis 5 oder hergestellt gemäss den Ansprüchen 6 oder 7 hergestellte oder modifizierte Polymere.

## Claims

1. A 1,4-diketopyrrolopyrrole of the formula 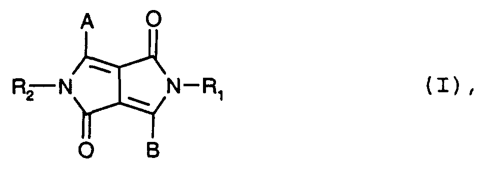 in which A and B independently of one another are a group of the formula 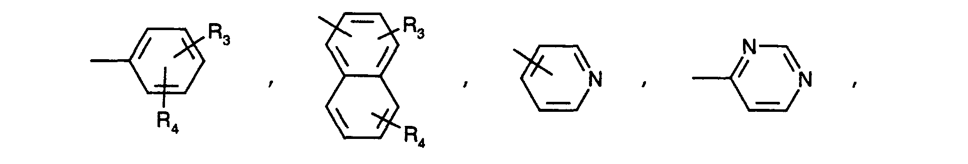 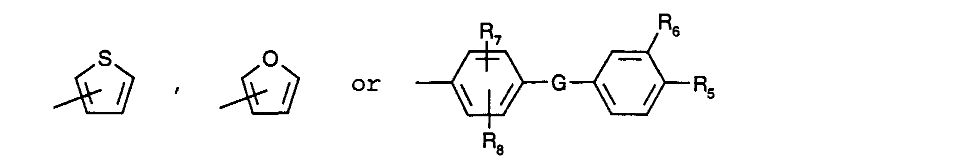 in which
R₃ and R₄ independently of one another are hydrogen, halogen, C₁-C₁₈alkyl,
C₁-C₁₈alkoxy, C₁-C₁₈alkylmercapto, C₁-C₁₈alkylamino, C₁-C₁₈alkoxycarbonyl,
C₁-C₁₈alkylaminocarbonyl, -CN, -NO₂, trifluoromethyl, C₅-C₆cycloalkyl, -C=N-(C₁-C₁₈alkyl), 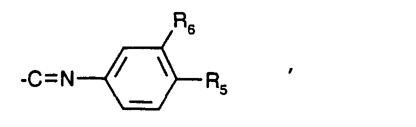 imidazolyl, pyrrazolyl, triazolyl, piperazinyl, pyrrolyl, oxazolyl, benzoxazolyl, benzothiazolyl, benzimidazolyl, morpholinyl, piperidinyl or pyrrolidinyl,
G is -CH₂-, -CH(CH₃)-, -C(CH₃)₂-, -CH=N-, -N=N-, -O-, -S-, -SO-, -SO₂-, -CONH- or -NR₉-,
R₅ and R₆ independently of one another are hydrogen, halogen, C₁-C₆alkyl, C₁-C₁₈alkoxy or -CN, R₇ and R₈ independently of one another are hydrogen, halogen or C₁-C₆alkyl and R₉ is hydrogen or C₁-C₆alkyl,
R₁ is a radical of the formula
- (CH₂)ₘ-CH=CH-(CH₂)ₙ-CH₃ (II)
or
-(Y)ₚ-X-(Z)ᵣ-Q (III)
in which m and n independently of one another are an integer between zero and 12, with the proviso that the sum m + n is at least 4, and p and r independently of one another are zero or 1, with the proviso that r is not zero if p is zero and Q is -OH or -COOR₁₁,
X is uninterrupted C₄-C₁₈alkylene or is C₄-C₁₈alkylene which is interrupted one or more times by -O- and/or -S-, -NH-, phenylene, -COO-, -CONH- or 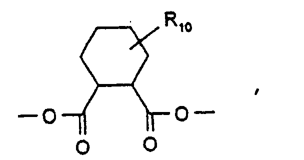 in which R₁₀ is hydrogen or methyl,
Y is 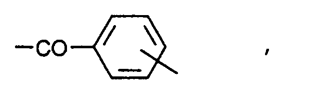 -Si(Cl)₂-, -Si(OC₂H₅)₂-, -Si(OCOCH₃)₂-, -CH₂-CH(OH)- or -CH(CN)- and Z is -O-, -NH-, -COO-, phenylene, 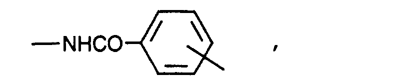 -Si(Cl)₂-, -Si(OC₂H₅)₂- or
-Si(OCOCH₃)₂-,
Q is -OH, -NH₂, glycidyl, 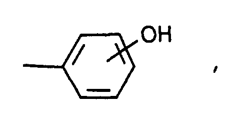 -CHO, -NCO, -CH=CH₂, -C(CH₃)=CH₂, -CO-CH=CH₂, -CO-C (CH₃) =CH₂, C₅-C₇cycloalkenyl, 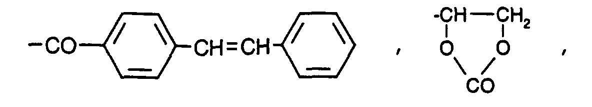 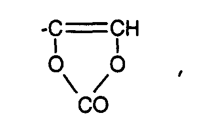 -CONHR₁₁, -COOR₁₁ or -COR₁₁, in which R₁₁ is hydrogen or C₁-C₆alkyl, or Q is 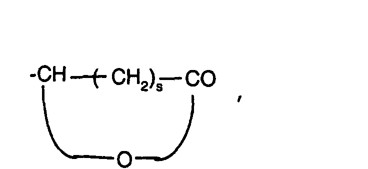 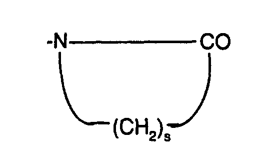 in which s is an integer from 1 to 6, and R₂ is C₁-C₆alkyl, 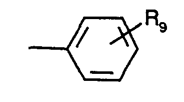 or R₁.

2. A diketopyrrolopyrrole according to claim 1, wherein, in the formula I, A and B independently of one another are a group of the formula 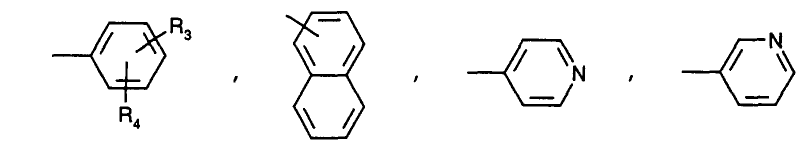 or 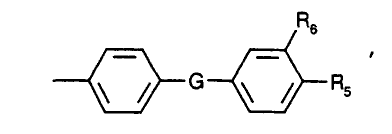 in which R₃ and R₄ independently of one another are hydrogen, halogen, in particular chlorine or bromine, C₁-C₄alkyl, C₁-C₆alkoxy, C₁-C₆alkylamino or CN,
G is -O-, -NR₉-, -N=N- or -SO₂-,
R₅ and R₆ are hydrogen, and R₉ is hydrogen, methyl or ethyl.

3. A diketopyrrolopyrrole according to claim 2, wherein, in the formula I, A and B are identical and are a group of the formula 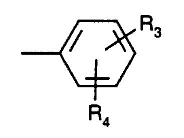 in which R₃ and R₄ independently of one another are hydrogen, methyl, tert-butyl, chlorine, bromine or CN.

4. A diketopyrrolopyrrole according to claim 1, wherein, in the formula I,
R₁ is a radical of the formula
-X-(O)ᵣ-Q (IV)
in which
X is uninterrupted C₄-C₁₂alkylene or is C₄-C₁₂alkylene which is interrupted 1, 2 or 3 times by O and/or once by -S-, -NH- or 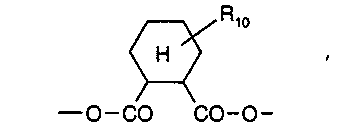 r and R₁₀ are as defined in claim 1, and
Q is -OH; -CH=CH₂, -C(CH₃)=CH₂, -CO-CH=CH₂ or -CO-C(CH₃)=CH₂.

5. A diketopyrrolopyrrole according to claim 4, wherein X is preferably -(CH₂)_{q}-, where q can be an integer between 6 and 12,
- (CH₂CH₂O)₂-CH₂CH₂-
or

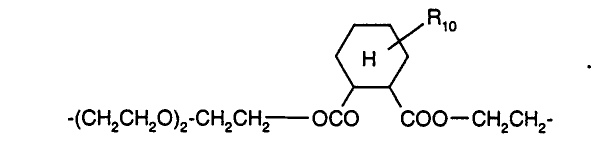

6. A process for preparing a diketopyrrolopyrrole according to claims 1 to 5 by reacting a pyrrolinone with a nitrile, which comprises (a) reacting a pyrrolinone of the formula 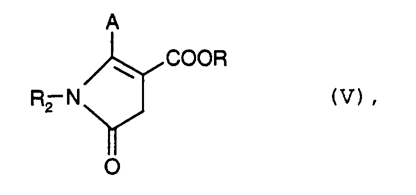 in which R is preferably C₁-C₄alkyl, with a nitrile of the formula
B-CN (VI),
where A and B are as defined in claim 1, R₁ is a radical of the formula II or III as defined in claim 2, and R₂ is C₁-C₆alkyl or 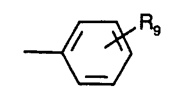 as defined in claim 1, and (a1) reacting the resulting diketopyrrolopyrrole with a compound of the formula
R₁-Hal (VII)
or, if Y in the radical with the formula III is, as defined in claim 2, -CH₂-CH(OH)-, with a compound of the formula 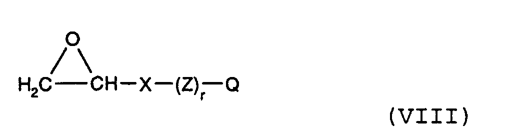 in which R₁, X, Z, Q and r are as defined in claim 2 and Hal is preferably chlorine or bromine, or (a2) reacting a diketopyrrolopyrrole of the formula 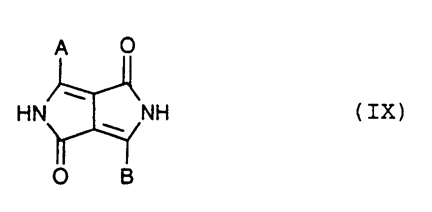 with the compounds of the formula VII or VIII, in a manner corresponding to that in (a1), or
(b) reacting a diketopyrrolopyrrole of the formula I in which R₁ is a radical of the formula III as defined in claim 2, where Q is -OH, with acryloyl chloride or methacryloyl chloride.

7. A method of preparing or modifiying polymers by a polymerization reaction or polymer-analogous reaction, which comprises polymerizing a diketopyrrolopyrrole of the formula I, in the presence if desired of an appropriate comonomer or of a polymer which carries polymerizable groups.

8. The use of a diketopyrrolopyrrole according to claims 1 to 5 or prepared according to claim 6 for preparing or modifying polymers by a polymerization reaction or a polymer-analogous reaction.

9. A polymer modified or prepared using a diketopyrrolopyrrole according to claims 1 to 5 or prepared according to claim 6 or 7.

## Revendications

1. 1,4-Dicétopyrrolopyrroles de formule 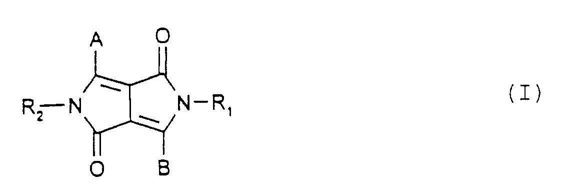 où A et B représentent, indépendamment l'un de l'autre, un groupe de formule 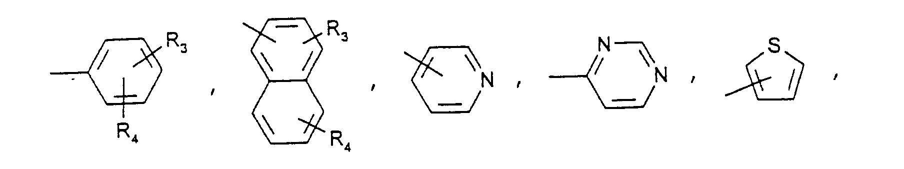 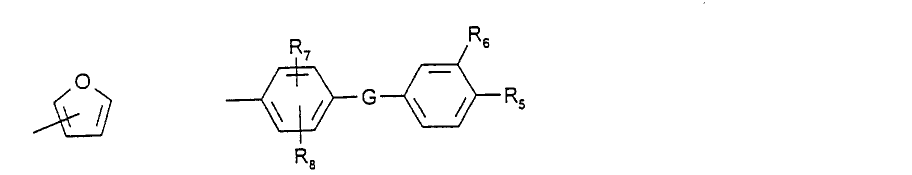 où
R₃ et R₄ représentent, indépendamment l'un de l'autre, des atomes d'hydrogène, des atomes d'halogènes, des groupes alkyle en C₁-C₁₈, alkoxy en C₁-C₁₈, (alkyl en C₁-C₁₈)-mercapto, (alkyl en C₁-C₁₈)amino, (alkoxy en C₁-C₁₈)-carbonyle, (alkyl en C₁-C₁₈)aminocarbonyle, -CN, -NO₂, trifluorométhyle, cycloalkyle en C₅-C₆, -C=N-(alkyle en C₁-C₁₈), 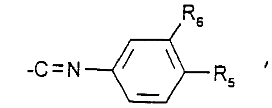 imidazolyle, pyrrazolyle, triazolyle, pipérazinyle, pyrrolyle, oxazolyle, benzoxazolyle, benzthiazolyle, benzimidazolyle, morpholinyle, pipéridinyle ou pyrrolidinyle,
G représente -CH₂-, -CH(CH₃)-, -C(CH₃)₂-, -CH=N-, -N=N-, -O-, -S-, -SO-, -SO₂-, -CONH- ou -NR₉-,
R₅ et R₆ représentent chacun, indépendamment l'un de l'autre, un atome d'hydrogène, un atome d'halogène, des groupes alkyle en C₁-C₆, alkoxy en C₁-C₁₈ ou -CN, R₇ et R₈ représentent, indépendamment l'un de l'autre, un atome d'hydrogène, un atome d'halogène ou un groupe alkyle en C₁-C₆ et R₉ un atome d'hydrogène ou un groupe alkyle en C₁-C₆,
R₁ représente un reste de formule
((CH₂)ₘ-CH=CH-(CH₂)ₙ-CH₃ (II)
-(Y)ₚ-X-(Z)ᵣ-Q (III)
où m et n sont, indépendamment l'un de l'autre, un entier compris entre zéro et 12, à condition que la somme de m+n soit d'au moins 4, et p et r valent, indépendamment l'un de l'autre, zéro ou 1, à condition que r ne soit pas égal à zéro, dans le cas où p est égal à zéro et Q représente des groupes -OH ou -COOR₁₁,
X représente un groupe alkylène en C₄-C₁₈ non interrompu ou interrompu une ou plusieurs fois par des groupes -O- et/ou -S-, -NH-, phénylène, -COO-, -CONH- 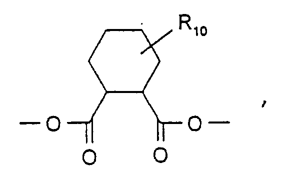 où R₁₀ représente un atome d'hydrogène ou un groupe méthyle,
Y représente des groupes 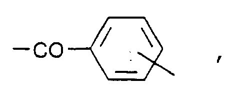 -Si(Cl)₂-, -Si(OC₂H₅)₂-, -Si(OCOCH₃)₂-, -CH₂CH(OH) ou CH(CN)- et
Z représente des groupes -O-, -NH-, -COO-, phénylène, 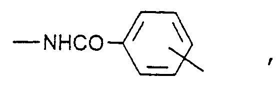 -Si(Cl)₂-, -Si(OC₂H₅)₂-, -Si(OCOCH₃)₂-,
Q représente des groupes -OH, -NH₂, glycidyle, 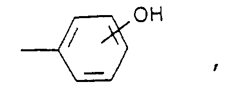 -CHO, -NCO, -CH=CH₂, -C-(CH₃)=CH₂, -CO-CH=CH₂, -CO-C(CH₃)=CH₂, cycloalcényle en C₅-C₇, 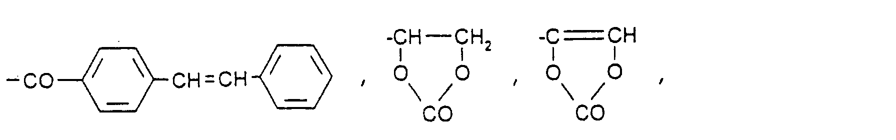 -CONHR₁₁, -COOR₁₁, -COR₁₁, où R₁₁ représente un atome d'hydrogène ou un groupe alkyle en C₁-C₆, 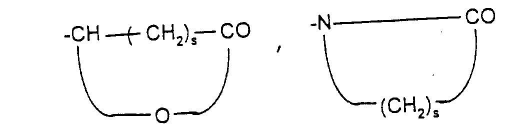 s étant un entier de 1 à 6, et
R₂ représente un groupe alkyle en C₁-C₆, 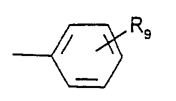 ou R₁.

2. Dicétopyrrolopyrroles selon la revendication 1, **caractérisés en ce que**, à la formule I, A et B représentent, indépendamment l'un de l'autre, un groupe de formule 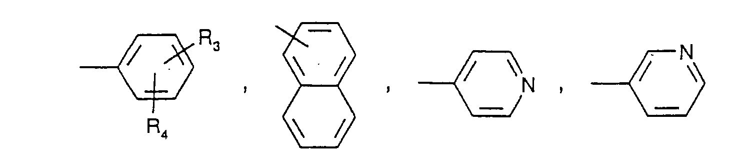 ou 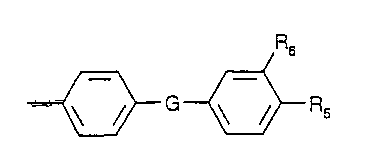 R₃ et R₄ représentent, indépendamment l'un de l'autre, des atomes d'hydrogène, des atomes d'halogènes, notamment le chlore ou le brome, des groupes alkyle en C₁-C₄, alkoxy en C₁-C₆, (alkyl en C₁-C₆)amino ou -CN,
G représente des groupes -O-, -NR₉, -N=N- ou -SO₂-,
R₅ et R₆ représentent des atomes d'hydrogène, R₉ représente un atome d'hydrogène, des groupes méthyle ou éthyle.

3. Dicétopyrrolopyrroles selon la revendication 2, **caractérisés en ce que**, à la formule I, A et B sont identiques et représentent un groupe de formule 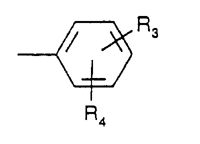 où R₃ et R₄ représentent, indépendamment l'un de l'autre, un atome d'hydrogène, des groupes méthyle, tert-butyle, chloro, bromo ou CN.

4. Dicétopyrrolopyrroles selon la revendication 1, **caractérisés en ce que**, à la formule I, R₁ représente un reste de formule
-X-(O)ᵣ-Q (IV)
où
X représente un groupe alkylène en C₄-C₁₂ non interrompu ou interrompu 1, 2 ou 3 fois par des groupes O et/ou une fois par des groupes -S-, -NH-, 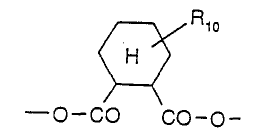 r et R₁₀ possèdent la signification donnée à la revendication 1, et
Q représente des groupes -OH; -CH=CH₂, -C(CH₃)=CH₂, -CO-CH=CH₂ ou -CO-C(CH₃)=CH₂

5. Dicétopyrrolopyrroles selon la revendication 4, **caractérisés en ce que** X représente de préférence un groupe -(CH₂)_{q}-, q pouvant être un entier compris entre 1 et 12, un groupe
- (CH₂CH₂O)₂-CH₂CH₂-
ou

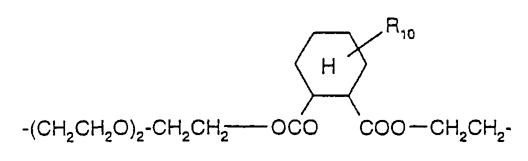

6. Procédé pour la préparation de dicétopyrrolopyrroles selon les revendications 1 à 5 par réaction d'une pyrrolinone sur un nitrile, **caractérisé en ce qu'**une pyrrolinone de formule 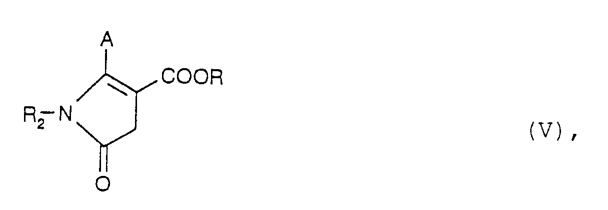 où R représente de préférence un groupe alkyle en C₁-C₄, réagit sur un nitrile de formule
B-CN (VI)
A et B possédant la signification donnée à la revendication 1, R₁ représente un reste de formule II ou III défini comme à la revendication 2 et R₂ représente un groupe alkyle en C₁-C₆ ou 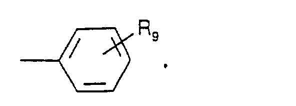 défini comme à la revendication 1, et (a1) on fait réagir le dicétopyrrolopyrrole obtenu sur un composé de formule
R₁-Hal (VII)
ou, lorsque Y dans le reste de formule III est défini comme à la revendication 2, représente un groupe -CH₂-CH-(OH)-, sur un composé de formule 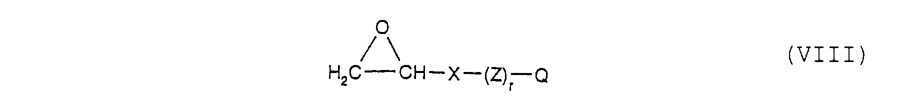 où R₁, X, Z, Q et r possèdent la signification donnée à la revendication 2 et Hal représente de préférence un atome de chlore ou de brome, ou (a2) on fait réagir un dicétopyrrolopyrrole de formule 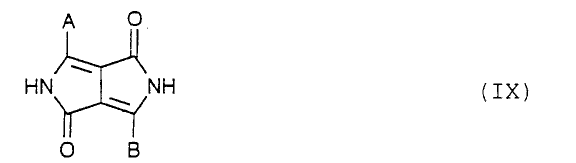 sur les composés de formules VII ou VIII de façon appropriée comme au point (a1), ou
(b) on fait réagir des dicétopyrrolopyrroles de formule I, où R₁ représente un reste de formule III avec Q = -OH comme défini comme à la revendication 2, sur un chlorure d'acide acrylique ou d'acide méthacrylique.

7. Procédé pour la préparation ou la modification de polymères par polyréaction ou une réaction analogue à la polymérisation, **caractérisé en ce qu'**on polymérise un dicétopyrrolopyrrole de formule I, si on le souhaite en présence d'un comonomère approprié ou d'un polymère porteur de groupes polyréactifs.

8. Utilisation de dicétopyrrolopyrroles selon les revendications 1 à 5 ou préparés selon la revendication 6, pour la préparation ou la modification de polymères par une polyréaction ou une réaction analogue à la polymérisation.

9. Polymères préparés à l'aide de dicétopyrrolopyrroles selon les revendications 1 à 5 ou préparés ou modifiés selon les revendications 6 ou 7.
